# EUROPÄISCHE PATENTSCHRIFT

(11) **EP 2 268 204 B1**
(45) Veröffentlichungstag und Bekanntmachung des Hinweises auf die Patenterteilung: **24.06.2020**
(21) Anmeldenummer: 09729759.2
(22) Anmeldetag: 09.04.2009
(51) Int. Cl.: G06T 11/00, A61B 6/03, G01N 23/04

(54) **VORRICHTUNG UND COMPUTER IMPLEMENTIERTES VERFAHREN ZUR ROTATIONSFREIEN COMPUTERTOMOGRAPHIE**
APPARATUS AND COMPUTER IMPLEMENTED METHOD FOR NON-ROTATIONAL COMPUTED TOMOGRAPHY
DISPOSITIF ET PROCÉDÉ MIS EN OEUVRE PAR ORDINATEUR POUR RÉALISER UNE TOMODENSITOMÉTRIE SANS ROTATION

(30) Priorität: 10.04.2008 DE 102008018269
(43) Veröffentlichungstag der Anmeldung: 05.01.2011
(73) Patentinhaber: Fraunhofer-Gesellschaft zur Förderung der angewandten Forschung e.V., 80686 München (DE)
(72) Erfinder: FUCHS, Theobald Dr., 90429 Nürnberg (DE); SCHÖN, Tobias, 90455 Nürnberg (DE); HANKE, Randolf, 90617 Puschendorf (DE)
(74) Vertreter: Zinkler, Franz
(86) Internationale Anmeldenummer: PCT/EP2009/002656
(87) Internationale Veröffentlichungsnummer: WO 2009/124774

(56) Entgegenhaltungen:
- WO-A-2007/047114

## Beschreibung

Ausführungsbeispiele der vorliegenden Erfindung befassen sich mit einer Vorrichtung bzw. einem Verfahren zum Erzeugen einer mehrdimensionalen Darstellung eines Objektes und dabei beispielsweise damit, wie Computertomographie-Bildrekonstruktionsverfahren angewendet werden können, ohne Rotationsbewegungen der Apparatur oder des zu untersuchenden Objekts in kleinen Winkelinkrementen durchzuführen.

Bei Computertomographieverfahren, die beispielsweise zur Diagnose am menschlichen Körper oder zur Materialprüfung im industriellen Bereich eingesetzt werden, wird üblicherweise ein zu untersuchendes Objekt von einer Röntgenquelle durchleuchtet, wobei ein Sensor, der bezogen auf das zu untersuchende Objekt gegenüber der Röntgenquelle angeordnet ist, Röntgenaufnahmen des durchleuchteten Objekts anfertigt. Bei Verwendung eines Flächensensors sind die so erzeugten Aufnahmen zweidimensional, bei Einsatz eines Zeilensensors eindimensional. Eine mehrdimensionale Darstellung, das heißt zum Beispiel eine dreidimensionale oder eine zweidimensionale Darstellung wird dadurch ermöglicht, dass die Perspektive, unter der das Objekt beleuchtet wird, bzw. unter der Aufnahmen des Objektes getätigt werden, verändert wird. Dabei werden unteren mehreren Perspektiven Aufnahmen des Objekts angefertigt. Durch die Veränderung der Perspektive, also beispielsweise des Winkels, unter dem das zu untersuchende Objekt beleuchtet wird, können aus den Projektionsaufnahmen Informationen über eine zusätzliche Dimension, nämlich Tiefeninformation, d.h. Ortsinformationen in einer Richtung zur Sensoroberfläche, gewonnen werden.

Bei diagnostischen Verfahren ist es dabei üblich, die Anordnung aus Röntgenquelle und Detektor um einen im Zentrum der Anordnung befindlichen Patienten zu rotieren. Bei Materialprüfungsverfahren wird oftmals auch das Objekt selbst rotiert, während die Anordnung aus Sensor und Röntgenquelle ortsfest bleibt. Um eine vollständige dreidimensionale Rekonstruktion bzw. mehrdimensionale Rekonstruktion des Objekts mit der maximalen zur Verfügung stehenden Ortsauflösung zu ermöglichen, ist es erforderlich, Projektionen des Objekts aus einem Raumwinkelbereich bzw. aus einem Flächenwinkelbereich von 180° in Parallelstrahlgeometrie aufzunehmen. Die Winkelinkremente benachbarter Aufnahmen hängen ebenso von der gewünschten erzielbaren Ortsauflösung, die hardwareseitig üblicherweise durch die Auflösung des verwendeten Sensors und die Präzision der Mechanik limitiert ist, abhängt.

Bei industriellen Materialprüfungsaufgaben sind die zu untersuchenden Objekte oftmals so groß, dass eine Rotation der Detektoranordnung um diese herum bzw. eine Rotation des Objekts selbst aus mechanischen oder Kostengründen nicht in Betracht kommt.

Selbst wenn eine Rotation möglich ist, wird bei der zerstörungsfreien Prüfung von Objekten mittels der rotationsabhängigen Computertomographieverfahren der 3D-Röntgencomputertomographie eine Begrenzung der erzielbaren Ortsauflösung häufig durch die Mechanik vorgegeben. Diese führt zu einer Verschlechterung der Ortsauflösung, die um einige Größenordnungen höher liegen kann, als die eigentliche erzielbare maximale Ortsauflösung, die durch die intrinsische Sensorauflösung des verwendeten Sensors limitiert wird. Dies rührt daher, dass für eine vollständige dreidimensionale Darstellung die Notwendigkeit besteht, das Objekt oder die Apparatur relativ zu diesem zu drehen, um Projektionen aus Richtungen von mindestens 180° + Flächenwinkel zu messen. Insbesondere bei großen Objekten (beispielsweise Frachtcontainern oder Frachtfahrzeugen mit Container) kann sich die mechanische Drehbewegung, selbst wenn diese prinzipiell möglich ist, schwierig gestalten, da große Gewichte bzw. große räumliche Dimensionen mit hoher Präzision und Wiederholungsgenauigkeit bewegt werden müssen. Dies stellt extrem hohe Anforderungen an die Justage und die Präzision der zur mechanischen Bewegung verwendeten Achsen.

Ähnliche Überlegungen gelten für Objekte mit ungünstiger, von Rotationssymmetrie stark abweichender Kontur bzw. Einhüllenden (wie beispielsweise Flugzeugflügel oder Leiterplatten). Bei solchen flachen, ausgedehnten Objekten kann der Abstand zwischen dem Brennfleck der Röntgenquelle und der Drehachse nicht beliebig klein gewählt werden, weil dieser mindestens so groß sein muss, dass das Objekt in seiner ungünstigsten geometrischen Ausdehnung an der Röntgenquelle vorbei rotiert bzw. vorbei bewegt werden kann. Dies resultiert in einer geometrisch verursachten Begrenzung der erzielbaren Auflösung, die durch eine optische Vergrößerung der abgebildeten Elemente des Objekts verbessert werden kann, indem die Quelle nahe an das zu untersuchende Objekt herangebracht wird, so dass nach den Gesetzen der Strahlenoptik eine vergrößerte Darstellung des Objekts auf dem Sensor abgebildet wird.

Die US-Patentanmeldung 6 814 489 B2 zeigt ein Rotations-CT, bei dem die Röntgenquelle und der Sensor radial bezüglich des zu untersuchenden Objekts verstellt werden können, um eine Vergrößerung in den Projektionsaufnahmen, die mittels der Apparatur erzeugt werden, einzustellen.

Die japanische Veröffentlichung "Study of New Linear Movement Tomography Using Magnification Ratio" beschreibt ein Verfahren, bei dem mehrere Röntgenaufnahmen eines zu untersuchenden Objekts getätigt werden, während sowohl Röntgenquelle als auch Röntgen-empfindlicher Sensor relativ zum untersuchenden Objekt bewegt werden. Nach einer Korrektur der Bilder auf die unterschiedlichen Vergrößerungsverhältnisse werden die so gewonnenen Bilder addiert, wobei sich durch die Addition ein Verwischungseffekt bezüglich derjenigen Bildelemente ergibt, die nicht in einer von der Verschiebung der Quelle und des Sensors abhängigen Ebene im Objekt liegen. Somit wird eine zwei-dimensionale Aufnahme durch einfache Addition erzeugt, bei dem die Konturen der Objekte in einer bevorzugten Ebene schärfer sind als die Konturen der Objekte in anderen Bildebenen.

Die US-Patentanmeldung 5 095 501 schlägt ein CT-Verfahren vor, bei dem zwei rechtwinklig zueinander angeordnete Paare von Röntgenquelle und Röntgendetektor verwendet werden, so dass die Untersuchungszeit des Patienten halbiert werden kann.

Die europäische Patentanmeldung 1 627 601 A1 befasst sich mit einem Verfahren, wie Streuartefakte, die durch metallische Implantate im Körper eines Patienten hervorgerufen werden, durch geeignete Bildverarbeitung unterdrückt werden können.

Die WO 2007/047114 A1 offenbart ein Abbildungssystem unter Verwendung einer Quelle und eines Detektors, wo die Quelle und das Objekt positioniert werden, so dass die Quelle in einem ersten Abstand von dem Objekt ist. Dann wird eine erste Projektion aufgezeichnet. Dann wird entweder die Quelle oder das Objekt derart bewegt, dass die Quelle an einem zweiten unterschiedlichen Abstand von dem Objekt ist. Hierauf wird ein zweites Projektionsbild unter Verwendung der Quelle und des Detektors aufgenommen.

Es besteht die Notwendigkeit für eine Vorrichtung bzw. für ein Verfahren, die es ermöglichen, Gegenstände bzw. Objekte zu untersuchen bzw. eine mehrdimensionale Darstellung dieser Objekte zu erzeugen, ohne das Objekt bezüglich der Detektoranordnung zu rotieren.

Dieser Notwendigkeit wird durch eine Vorrichtung zum Erzeugen einer mehrdimensionalen Darstellung eines Objekts nach Patentanspruch 1, ein Verfahren zum Erzeugen einer mehrdimensionalen Darstellung eines Objekts, oder ein Computer-Programm nach Patentanspruch 15 entsprochen.

Einige Ausführungsbeispiele der Erfindung ermöglichen es, eine mehrdimensionale Darstellung des Objekts zu erhalten, indem das ortsfeste Objekt mittels einer oder mehrerer Translationsbewegungen der Röntgenquelle abgebildet wird. Der Detektor bzw. der Sensor kann bezüglich der Röntgenquelle bzw. der Quelle räumlich auf verschiedene Art und Weise angeordnet sein. Um eine Abbildung auf dem Sensor bzw. dem Detektor zu ermöglichen, befindet sich dieser im Strahlengang hinter dem zu untersuchenden Objekt. Bei einigen Ausführungsbeispielen der Erfindung erfolgen die Translationsbewegungen in einer Richtung senkrecht zur Oberfläche des verwendeten Sensors bzw. Detektors. Im Fall eines zweidimensionalen Detektors also in Richtung der Oberflächennormalen und im Falle eines Zeilendetektors senkrecht zur Zeilenrichtung, in Richtung der Oberflächennormalen der Pixel, die in der Zeile verwendet werden. Bei weiteren Ausführungsbeispielen wird die Bewegung nicht exakt senkrecht zur Oberfläche des Sensors durchgeführt, sondern eine Bewegung wird in einer Vorzugsrichtung durchgeführt, die sich aus einer relativen Position des Objekts zum Sensor bestimmt. Beispielsweise kann die Vorzugsrichtung einen Winkel zur Flächennormale der Sensorelemente aufweisen, wie beispielsweise 22,5 oder 45 Grad.

Bei einigen Ausführungsbeispielen der Erfindung besteht die Vorrichtung zum Erzeugen einer mehrdimensionalen Darstellung aus einer Röntgenquelle, die zur Beleuchtung des Objekts verwendet wird, aus einem Sensor zur Aufnahme eines Projektionsbildes des Objekts und aus einer Bewegungseinrichtung, die das Objekt oder die Quelle bewegen kann, um einen Abstand in der Vorzugsrichtung zwischen der Röntgenquelle und dem Objekt zu verändern, so dass eine erste und eine zweite Aufnahme des Objekts bei unterschiedlichen Abständen angefertigt werden kann. Der Sensor befindet sich bezüglich der Röntgenquelle in einer durch die relative Position des Objekts und des Sensors gegebenen Vorzugsrichtung hinter dem Objekt. Indem der Abstand zwischen der Röntgenquelle und dem Objekt verändert wird, wird die Perspektive, unter der das Objekt aufgenommen wird, geändert. Dies erlaubt es, ohne Rotation unterschiedliche Perspektiven des Objekts aufzunehmen, um beispielsweise bei Verwendung eines zweidimensionalen Sensors durch Kombination der Aufnahmen eine dreidimensionale Darstellung des zu untersuchenden Objekts zu erhalten.

Bei einigen Ausführungsbeispielen der Erfindung werden zwei unabhängig voneinander bewegbare Röntgenquellen verwendet, die in derselben Vorzugsrichtung bewegt werden können, jedoch in einer Richtung senkrecht zur Vorzugsrichtung (der Bewegungsrichtung der Röntgenquellen) räumlich voneinander getrennt angeordnet sind. Durch die Anordnung kann durch schrittweises Bewegen der beiden Röntgenquellen erreicht werden, dass das Objekt in seiner Vollständigkeit bzw. besonders interessante Teilbereiche des Objekts aus Perspektiven aufgenommen bzw. abgebildet werden, die einen Winkelbereich von bis zu 180° überspannen.

Bei weiteren alternativen Ausführungsbeispielen wird dies ermöglicht, indem zwei Detektoren und zwei Röntgenquellen verwendet werden, wobei die Detektoren und die Röntgenquellen so angeordnet sind, dass diese zueinander in einem rechten Winkel angeordnete Vorzugsrichtung aufweisen. Dadurch kann ebenfalls eine Abbildung des Objekts aus Perspektiven, die einen Winkelbereich von bis zu 180° umfassen, ermöglicht werden.

Bei weiteren Ausführungsbeispielen der Erfindung kann die Vorzugsrichtung relativ zum zu untersuchenden Objekt in vorgegebenen oder frei wählbaren Winkelinkrementen verändert werden, wobei je Vorzugsrichtung eine Mehrzahl von Röntgenaufnahmen mit unterschiedlichen Abständen des Objekts zur Röntgenquelle (Abstand in der Vorzugsrichtung) durchgeführt wird. Zu diesem Zweck kann beispielsweise eine bewegliche Achse oder eine Bewegungseinrichtung, auf der die Röntgenquelle angeordnet ist, und auf der die Röntgenquelle in Richtung des zu untersuchenden Objekts bewegt werden kann, drehbar gelagert sein. Dies erhöht die Ortsauflösung zusätzlich, indem der überspannte Winkelbereich in große Winkelinkremente, die durch ein Verkippen der Achse erzielt werden, und in kleine Winkelinkremente, die durch ein Bewegen der Röntgenquelle in Richtung der Vorzugsrichtung erreicht werden, aufgeteilt werden. Dadurch ist es möglich, beispielsweise an die Oberfläche eines ausgedehnten, ungleichmäßig geformten Objekts mit der Röntgenquelle nahe heran zu gehen, um eine hohe Auflösung zu erzielen, während gleichzeitig sichergestellt ist, dass die Aufnahme mit einer Vielzahl kleiner Winkelinkremente durchgeführt werden kann, was zur Erzielung einer hohen Ortsauflösung ebenfalls erforderlich ist.

Bei weiteren Ausführungsbeispielen kann das zu untersuchende Objekt in einer Richtung senkrecht zur Vorzugsrichtung bzw. parallel zur Sensoroberfläche an der Vorrichtung aus Sensor und Röntgenquelle vorbeigeführt werden, um so auch bei äußerst ausgedehnten zu untersuchenden Objekten zu ermöglichen, eine dreidimensionale bzw. mehrdimensionale Rekonstruktion des gesamten Volumens des zu untersuchenden Objekts zu erhalten. Bei alternativen Ausführungsbeispielen wird nicht das Objekt am Detektor bzw. an der Vorrichtung vorbei bewegt, sondern das feststehende Objekt wird mit einem mobilen Detektor abgefahren, so dass also die Vorrichtung bewegt wird, während das Objekt selbst ortsfest bleibt.

Nachfolgend werden, bezugnehmend auf die beigefügten Figuren, einige Ausführungsbeispiele der vorliegenden Erfindung detaillierter beschrieben. Es zeigen:
- Fig. 1: ein Beispiel für eine Vorrichtung zum Erzeugen einer mehrdimensionalen Darstellung eines Objekts;
- Fig. 2: ein weiteres Beispiel für eine Vorrichtung zum Erzeugen einer mehrdimensionalen Darstellung;
- Fig. 3: ein weiteres Ausführungsbeispiel einer Vorrichtung zum Erzeugen einer mehrdimensionalen Darstellung mit einer Röntgenquelle an verschiedenen Positionen;
- Fig. 4: ein weiteres Ausführungsbeispiel einer Vorrichtung zum Erzeugen einer mehrdimensionalen Darstellung mit zwei orthogonal zueinander verlaufenden Achsen möglicher Röntgenquellenpositionen;
- Fig. 5: ein weiteres Ausführungsbeispiel einer Vorrichtung zum Erzeugen einer mehrdimensionalen Darstellung mit zwei parallel verlaufenden Achsen möglicher Röntgenquellenpositionen;
- Fig. 6: ein weiteres Ausführungsbeispiel einer Vorrichtung zum Erzeugen einer mehrdimensionalen Darstellung mit sich in lateraler Richtung unterscheidenden Objekt- oder Quellenpositionen;
- Fig. 7: ein Ausführungsbeispiel mit rotierbar angebrachter Röntgenquelle;
- Fig. 8: ein Beispiel eines Systems zum Erzeugen einer dreidimensionalen Darstellung eines ausgedehnten Objekts;
- Fig. 9: ein Beispiel für die Vermessung eines ortsfesten Rohres; und
- Fig. 10: ein weiteres Beispiel für die Vermessung ortsfester Objekte.

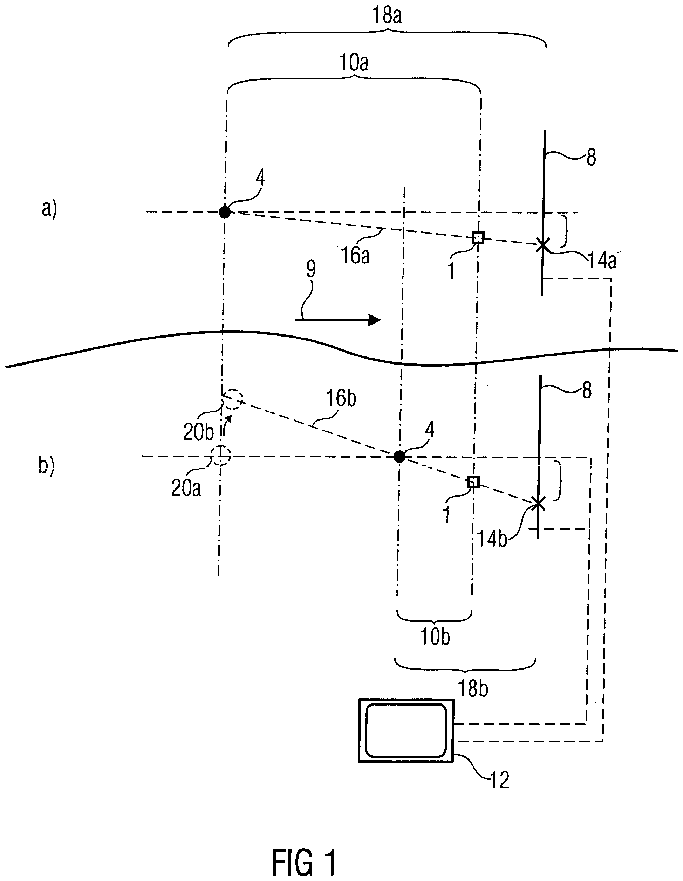

Fig. 1 zeigt schematisch ein Beispiel einer Vorrichtung zum Erzeugen einer mehrdimensionalen Darstellung eines Objekts 1. Das Objekt 1, das sich an beliebiger Position innerhalb eines beobachtbaren Messfeldes 2 befinden kann, wird mittels einer Röntgenquelle 4 beleuchtet. Ein Sensor 8 befindet sich bezüglich einer Vorzugsrichtung 9 hinter dem Objekt 1, so dass dieser, wenn das Objekt 1 mit der Röntgenquelle 4 beleuchtet wird, dazu verwendet werden kann, eine zweidimensionale bzw. eine eindimensionale (falls ein Zeilensensor verwendet wird) Aufnahme bzw. Darstellung des Objekts aufzunehmen.

Die Vorzugsrichtung wird durch die relative Position des Objekts 1 und des Sensors 8 gegeben und ist im in Fig. 1 gezeigten Fall senkrecht zur Sensoroberfläche des Sensors 8, also parallel zur Flächennormale der Sensorelemente des Sensors 8. Der Sensor 8 kann dabei beispielsweise ein Matrixsensor oder ein Zeilensensor sein, der auf Röntgenstrahlung sensitiv ist, so wie beispielsweise mit Phosphor beschichtete CCD-Elemente, Photomultiplierarrays oder dergleichen.

Fig. 1 zeigt oben (a) eine Konfiguration, bei der die Röntgenquelle 4 in einem ersten Abstand 10a zum Objekt 1 angeordnet ist, wo hingegen die zweite Darstellung (b) eine Konfiguration zeigt, in der die Röntgenquelle 4 in einem zweiten Abstand (10b) zum Objekt 1 angeordnet ist.

Die Fig. 1a und 1b zeigen zwei Konfigurationen, mittels derer die zweidimensionale Position des Objekts 1 im Raum bestimmt werden kann, wenn bei ortsfestem Sensor 8 eine erste und eine zweite Aufnahme des Objekts gemacht werden. Werden beide Aufnahmen mittels eines Computers bzw. einer geeigneten Kombinationseinrichtung 12 ausgewertet, lässt sich auf die im Folgenden kurz dargelegten Weise die Position des Objekts 1 im Raum bestimmen. Zur Vereinfachung der Darstellung wird dabei davon ausgegangen, dass das Objekt 1 eine vernachlässigbar kleine räumliche Ausdehnung hat, so dass die inneren Strukturen des Objekts 1 nicht aufgelöst werden müssen. Vielmehr ist die vollständige Information über das Objekt 1 erlangt, sofern dessen Position in Vorzugsrichtung 9 und dessen Position parallel zur Sensoroberfläche 8 bekannt ist. Es sei an dieser Stelle angemerkt, dass die Überlegungen zwanglos auf ein ausgedehntes dreidimensionales bzw. zweidimensionales Objekt verallgemeinert werden können, wenn jedes Volumen oder jedes ortsauflösbare Volumen innerhalb des ausgedehnten Objekts dem im Folgenden diskutierten Objekt 1 gleichgesetzt wird.

Das anhand der Fig. 1a und 1b beschriebene zugrundeliegende Konzept zur Aufnahme von zwei Projektionen eines Objekts 1 bei unterschiedlichen Röntgenquellenpositionen, die durch eine Translationsbewegung auseinander hervorgehen, lässt sich auf Standard-Computertomographie-Algorithmen zur Bildauswertung übertragen, um so ortsaufgelöste zweidimensionale oder dreidimensionale Schnittaufnahmen bzw. Darstellungen von zu untersuchenden Objekten zu erhalten.

In der in Fig. 1a dargestellten Konfiguration wird von der Röntgenquelle 4 eine Projektion des Objekts 1 an der Position 14a auf der Oberfläche des Sensor 8 erzeugt. Ist die Geometrie der Anordnung bekannt, das heißt, der Abstand von Röntgenquelle 4 und Sensor 8, lässt sich durch einfache geometrische Überlegungen der "Röntgenstrahl" 16a rekonstruieren, auf dem sich das Objekt 1 befinden muss. Aufgrund der Projektion der Darstellung ist jedoch nicht erkennbar, wo auf dem Röntgenstrahl 16a das Objekt 1 befindlich ist, wenn lediglich eine Aufnahme durchgeführt wird.

Selbiges gilt, wenn mit der in Fig. 1b gezeigten Konfiguration eine Aufnahme getätigt wird, die eine Projektion des Objekts 1 an Position 14b auf der Oberfläche des Sensors 8 erzeugt. Auch hier ist zunächst nur die Rekonstruktion eines Röntgenstrahls 16b möglich, auf dem sich das Objekt 1 befinden muss.

Kennt man jedoch die Geometrie der Apparatur und insbesondere die Abstände 18a und 18b zwischen Röntgenquelle und Röntgensensor bzw. die Positionen 14a und 14b auf der Oberfläche des Sensors 8, lässt sich die Information, an welcher Position in Vorzugsrichtung 9 sich das Objekt auf den jeweiligen Röntgenstrahlen befindet, rekonstruieren. Auch geometrisch ist diese Rekonstruktion möglich, wenn die beiden Röntgenstrahlen zum Schnitt gebracht werden.

An dem Schnittpunkt befindet sich das Objekt im Raum. Wie gesagt, lässt sich diese Betrachtung auch auf ausgedehnte Objekte verallgemeinern, die sich vor dem Sensor 8 befinden mögen. Im Fall einer zweidimensionalen Aufnahme ist die dreidimensionale Rekonstruktion ebenso möglich, indem aus den zweidimensionalen Projektion die Tiefeninformation auf die oben beschriebene Art und Weise gewonnen wird.

Durch die Kombination der Aufnahmen mit den unterschiedlichen Abständen zwischen Röntgenquelle 4 und Objekt 1 lässt sich also eine mehrdimensionale Darstellung des Objekts 1 erzeugen.

Dabei lassen sich bei ausgedehnten Objekten insbesondere auch aus der Computertomographie bekannte Algorithmen bzw. Bildrekonstruktionsverfahren verwenden, da, wie in Fig. 1b skizziert, die Translation der Röntgenquelle 4, um dem Abstand 10a in den Abstand 10b zu ändern, einer Rotation der Röntgenquelle 4 aus Fig. 1a um die neue Position der Röntgenquelle 4 in Fig. 1b äquivalent ist.

Mit anderen Worten kann eine durch die in Fig. 1 beschriebene Translation der Röntgenquelle hervorgerufene Änderung der Perspektive, unter der Objekt 2 beleuchtet wird, einer Rotation der Röntgenquelle 4 gleichgesetzt werden, wie sie aus herkömmlichen CT-Verfahren bekannt ist.

Aus der Translation von Fig. 1a nach 1b lässt sich insbesondere die dazu äquivalente Rotationsbewegung der Röntgenquelle 4 von der Position 20a in die virtuelle Position 20b ableiten.

Bei Kenntnis der Rotationsbewegung bzw. des Rotationszentrums lassen sich somit CT-Algorithmen zur Berechnung der dreidimensionalen bzw. mehrdimensionalen Darstellung eines Objekts unmittelbar anwenden, wobei auf die Notwendigkeit, entweder den gesamten Detektoraufbau oder das Objekt zu rotieren, verzichtet werden kann. Dies hat die weiter oben beschriebenen Vorteile, insbesondere bei der Untersuchung großer, ausgedehnter Objekte hoher Masse, bei denen eine Rotation entweder aufgrund ihrer geometrischen Ausdehnung nicht möglich ist oder eine durch die Masse der Objekte verursachte mechanische Ungenauigkeit der Steuerung der Rotationswinkel zu einer großen Verschlechterung der Auflösung der CT-Aufnahmen führen würde.

Anhand der in Fig. 1 gezeigten Prinzipskizze versteht sich von selbst, dass die Rekonstruktionsalgorithmen, die in der Kombinationseinrichtung verwendet werden, nicht notwendigerweise als intermediären Schritt eine Umwandlung der Translationsbewegung der Röntgenquelle 4 in die in Fig. 1b skizzierte Rotationsbewegung umfassen müssen. Vielmehr ist es selbstverständlich auch möglich, die CT-Analysealgorithmen umzuformulieren, um unmittelbar aus den ausgenommenen Aufnahmen des Sensors 8 bei Kenntnis der Translationsbewegung der Röntgenquelle die mehrdimensionale Darstellung zu extrahieren bzw. abzuleiten.

Es sei ferner angemerkt, dass nicht notwendigerweise die Röntgenquelle 4 bewegt werden muss, um zu der Änderung der Perspektive der Abbildung zu gelangen, sondern dass statt dessen ebenso das Objekt 1 selbst in der Vorzugsrichtung von der Röntgenquelle 4 weg oder auf diese zu bewegt werden kann. Dies führt, ebenso wie das in Fig. 1 skizzierte Bewegen der Röntgenquelle zu einer Änderung der Perspektive und erlaubt somit die Tiefeninformation bzw. die Ortsinformation in Richtung der Vorzugsrichtung 9 aus den Aufnahmen zu extrahieren, um somit zu einer mehrdimensionalen Darstellung des Objekts zu gelangen.

Mit anderen Worten illustriert Fig. 1 schematisch das Prinzip, auf Basis von einer oder mehrerer linearer Verschiebungen bzw. Translationsbewegungen der Röntgenquelle 4 (oder des Objekts 1) unter weitest gehendem Verzicht von Drehbewegungen des Objekts oder der Apparatur das Volumen eines Objekts 1 in Anlehnung an herkömmliche Verfahren der Computertomographie dreidimensional und vollständig zu rekonstruieren. Dies ist, wie in Fig. 1 dargestellt, möglich, da sich bei einer Veränderung des senkrechten Abstands zur Röntgenquelle zum Objekt während der Aufnahme mehrerer Projektionen die Perspektive verändert, so dass Informationen über den inneren Aufbau des Objekts gewonnen werden können, also insbesondere Tiefeninformationen zugänglich werden. Dies kann auch als Translations-CT bezeichnet werden, da das Vorgehen dem einer CT äquivalent ist, diese Untersuchung jedoch ohne Rotationsbewegungen auskommt.

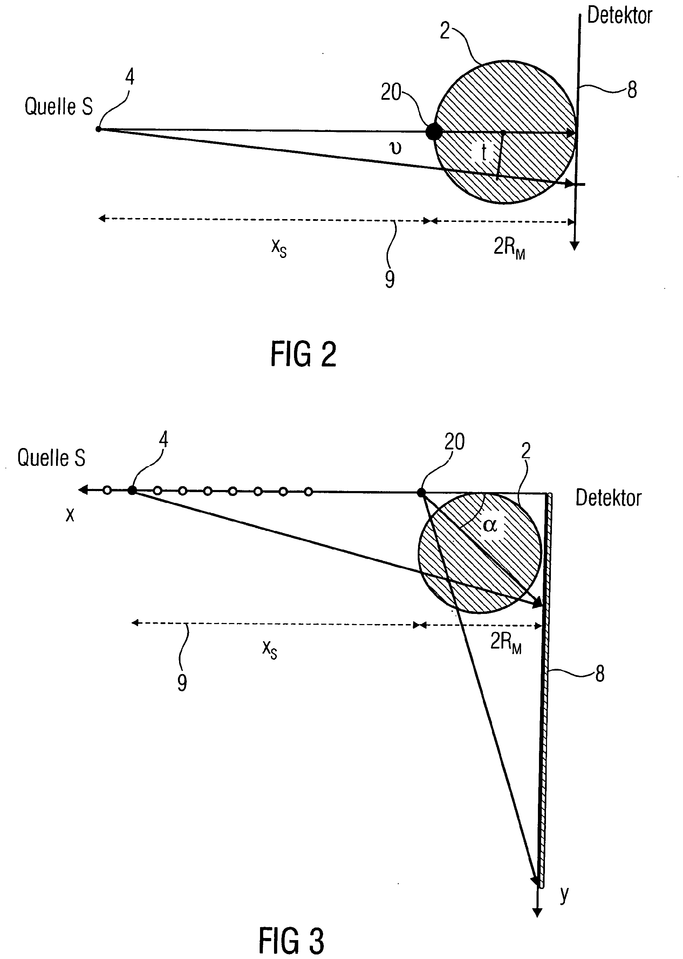

Fig. 2 zeigt ein weiteres Ausführungsbeispiel einer Vorrichtung zum Erzeugen einer mehrdimensionalen Darstellung eines Objekts, bei dem ein Objekt innerhalb eines Messfeldes 2 von einer Röntgenquelle 4 auf einen Detektor 8 abgebildet wird, wobei die Röntgenquelle 4 in einer zur Oberfläche des Detektors bzw. Sensors 8 senkrechten Richtung bewegt werden kann. Bei dem in Fig. 2 dargestellten Fall wird ein ausgedehntes Messfeld 2 betrachtet, auf das sich, wie oben bereits beschrieben, die anhand der Fig. 1 beschriebenen Überlegungen übertragen lassen.

Dabei wird die Quelle 4 in einer Vorzugsrichtung 9 auf den Sensor 8 zu bzw. von dem Sensor 8 (Detektor) wegbewegt, um eine Mehrzahl von Aufnahmen zu erzeugen, die mittels einer hier nicht dargestellten Kombinationseinrichtung bzw. einem Auswertecomputer ausgewertet werden können, um zu der mehrdimensionalen Darstellung des Messfelds 2 zu gelangen.

Dabei zeigt Fig. 2 eine mögliche Anordnung, die symmetrisch ist, bei der also der Zentralstrahl einer vereinfachend als punktförmig angenommenen Röntgenquelle 4 den Detektor mittig trifft. Das heißt, derjenige Strahl, der senkrecht auf die Detektoroberfläche trifft, trifft diese in der Mitte. Ein Messdatensatz wird beispielsweise gewonnen, indem das Projektionsbild des Messfelds 2 auf dem Sensor 8 aus einer Vielzahl von Quellpositionen (und damit Perspektiven), die sich durch den Abstand xₛ zum Messfeld 2 unterscheiden, aufgezeichnet werden. Das Messfeld 2 muss dabei selbstverständlich nicht kugelsymmetrisch bzw. kreisförmig sein, wie es in Fig. 2 dargestellt ist. Das in Fig. 2 dargestellte Messfeld 2 dient lediglich als Definition desjenigen Raumbereichs, welcher überhaupt mit der Anordnung von Fig. 2 bearbeitet werden kann. Innerhalb dieses Messfeldes 2 sind beliebige geometrische Anordnungen bzw. Objekte denkbar. Somit definiert das Messfeld von Fig. 2 und den folgenden dargestellten Figuren lediglich diejenige Fläche, innerhalb derer eine Messung mit den jeweils betrachteten Vorrichtungen sinnvoll durchgeführt werden kann.

In anderen Worten wird in Fig. 2 ebenso wie in den nachfolgend diskutierten Ausführungsbeispielen ein zweidimensionales Messfeld angedeutet. In der Richtung senkrecht zur Papierebene kann das Verfahren bzw. die Vorrichtung, die hier diskutiert wird, jedoch einfach ergänzt werden, wobei beispielsweise Daten, die für eine dreidimensionale Rekonstruktion benötigt werden, von einem geeigneten Zeilen- oder Flachbilddetektor aufgezeichnet werden. Werden einzelne Sensorzeilen als voneinander vollständig unabhängig betrachtet, können die hier im für den eindimensionalen Fall diskutierten Ausführungsbeispiele unmittelbar auf den zweidimensionalen Sensor angewendet werden. In dieser analogen Betrachtungsweise liefern die voneinander unabhängigen Zeilen Daten für entsprechende Schichten durch das Objekt.

Auch eine Adaption anderer Volumen-CT-Verfahren an das Prinzip der linearen Translationsbewegung der Röntgenquelle bzw. an das Prinzip der Abstandsveränderung zur Erfassung eines wesentlichen Datenbereichs für eine dreidimensionale Rekonstruktion ist selbstverständlich möglich.

Dabei kann selbstverständlich auch eine zwei-dimensionale Rekonstruktion basierend auf einem eindimensionalen Sensor erfolgen.

Basierend auf den in unterschiedlichen Abständen zwischen Objekt und Röntgenquelle in der Vorzugsrichtung gewonnenen Aufnahmen erfolgt eine Filterung, mittels derer eine mehrdimensionale Rekonstruktion des untersuchten Objekts erfolgt. Die Filterung wird beispielsweise von einer Kombinationseinrichtung vorgenommen, die auf die so erzeugten Aufnahmen einen Computertomographie-Bildverarbeitungsalgorithmus anwendet, um die mehrdimensionale Rekonstruktion zu erhalten. Dieser Algorithmus kann beispielsweise eine Radon-Transformation umfassen. Dabei wird bei einigen Ausführungsbeispielen der Erfindung aus der Translationsbewegung in der Vorzugsrichtung, die durch die Bewegungseinrichtung hervorgerufen wird, ein Rotationswinkel abgeleitet, der zu der Perspektivenänderung korrespondiert, die sich bezüglich des abgebildeten Objekts auf dem Sensor durch die Translation ergibt. Gemäß einigen Ausführungsbeispielen ist die Bewegungseinrichtung derart ausgeführt, dass durch eine einzelne Translationsbewegung jeweils ein vorbestimmtes festes Winkelinkrement des Rotationswinkels erzeugt wird. Bei anderen Ausführungsbeispielen können die Inkremente der Rotationswinkel selbstverständlich beliebig sein. Das heißt, eine Serie aus erster Aufnahme, zweiter Aufnahme und mehreren weiteren Aufnahmen kann entweder mit beliebigen Winkelinkrementen als auch mit äquidistanten Winkelinkrementen angefertigt werden, um daraus durch Anwendung eines Computertomographie-Bildverarbeitungsalgorithmus eine mehrdimensionale Rekonstruktion des Objekts zu erhalten.

Bei einigen Ausführungsbeispielen der Erfindung weist die Vorrichtung zum Erzeugen einer mehrdimensionalen Darstellung also ferner eine Sortiereinrichtung auf, die die getätigten Aufnahmen einer Serie (zumindest der ersten und der zweiten Aufnahme) von einer Rekonstruktion zugrunde liegenden Aufnahmen derart umsortiert, dass sie in monoton steigender oder fallender Folge nach Winkelinkrementen sortiert vorliegen, um die so aufbereitet einem Computertomographie-Bildverarbeitungsalgorithmus zur Verfügung zu stellen.

Wie bereits oben erwähnt, ermöglicht das Verfahren bzw. die Vorrichtung zur Erzeugung einer mehrdimensionalen Darstellung durch die Translationsbewegung der Röntgenquelle bzw. das Verändern des Abstands zwischen Röntgenquelle 4 und Objekt (bzw. Messfeld 2), auch bei ausgedehnten Objekten, die Darstellungen mit der jeweils maximalen möglichen, durch die Dicke des beobachteten Objekts limitierten, Vergrößerung (Auflösung) zu erzeugen. Dies ist deshalb der Fall, da lokal an das zu untersuchende Objekt mit der Röntgenquelle nahe herangegangen werden kann, weil die Röntgenquelle, falls ein dickerer Teil des zu untersuchenden makroskopischen Objekts zu untersuchen ist, einfach wieder von dem Objekt weg bewegt werden kann. Aufgrund der geometrischen Vergrößerung der Abbildung auf dem Sensor 8, kann somit für dünnere Objekte eine höhere Ortsauflösung erzielt werden. Dies ist bei solchen Objekten mit auf Rotation basierenden Verfahren bzw. Vorrichtungen unmöglich, da dort die der Abstand der Röntgenquelle zum Sensor immer durch die maximale geometrische Ausdehnung des zu untersuchenden Objekts bestimmt wird.

Im in Fig. 2 gezeigten Fall und auch in den weiteren beschriebenen Ausführungsbeispielen werden die Daten also durch angepasste Algorithmen der gefilterten Rückprojektion bzw. die dazu korrespondierenden algebraischen Verfahren rekonstruiert. Da mit dem vorgeschlagenen Prinzip sogar so hohe Vergrößerungen erzielt werden können, dass nicht mehr sämtliche Bereiche des zu untersuchenden Messfelds 2 auf dem Sensor abgebildet werden können (wenn sich die Röntgenquelle 4 unmittelbar vor dem Messfeld 2 befindet) können hinsichtlich des dann unvollständigen Datenbereichs Methoden zur Datenergänzung bzw. Extrapolation und andere Rekonstruktionsverfahren für den stark vergrößerten Bereich (ROI = region of interest) direkt oder ggf. nach einer Anpassung an die geometrischen Gegebenheiten zum Einsatz kommen (beispielsweise basierend auf einer Wavelet- oder Hilbert-Transformation) .

Die verfügbaren Rekonstruktionsverfahren lassen sich dabei zunächst in algebraische Rekonstruktionsverfahren und analytische Rekonstruktionsverfahren unterscheiden, wobei bei den analytischen Rekonstruktionsverfahren die geometrischen Rahmenbedingungen, also beispielsweise der Abstand der Röntgenquelle und des Objekts vom Sensor, bekannt sein müssen. Bei einer herkömmlichen Art der Rekonstruktion ist ein vollständiger Datensatz insofern erforderlich, als dass das Objekt vollständig aus einem Raumwinkelbereich von 180° mittels paralleler Röntgenstrahlen beleuchtet werden muss, um aus den so erzeugten Aufnahmen eine vollständige Bildrekonstruktion zu ermöglichen. Dies ist insbesondere deshalb der Fall, da bei diesem Standard-Verfahren auf die Daten jeweils globale Operationen, die auf das gesamte Objekt wirken, verwendet werden. Durch gefilterte Rückprojektion lässt sich bei vollständigem Datensatz die mehrdimensionale Aufnahme des Objektes rekonstruieren.

Wenn die oben angesprochenen (Projektions-) Daten nicht vollständig zur Verfügung stehen oder Teile dieser Daten nicht verwendbar sind, können die Datensätze durch Extra- oder Interpolation vervollständigt werden, um zu einem vollständigen Datensatz zu gelangen, der als Grundlage für die Anwendung des Standard- Rekonstruktionsverfahrens verwendet werden kann.

Neuere Verfahren erlauben es, auch ohne Extra- oder Interpolation durch Anwendung lokaler Operationen auf unvollständigen Datensätzen Teilbereiche des zu untersuchenden Objektes zu rekonstruieren. Zur Analyse werden dann orts- und frequenz-lokale Funktionen verwendet, wie beispielsweise Wavelets. Weitere Verfahren, die auf unvollständigen Datensätzen operieren können, benutzen die Differentiale der rückprojezierten Daten und eine auf der Hilbert-Transformation des Bildes entlang spezieller Röntgenlinien basierenden Darstellung, um das Objekt in der ROI zu rekonstruieren. Allen diesen Verfahren ist gemein, dass die geometrischen Randbedingungen bekannt sein müssen, also dass bekannt ist, wie die Röntgenstrahlen (idealisiert) infinitesimaler Ausdehnung durch das Objekt verlaufen. Solche Röntgenlinien bzw. -strahlen werden dabei beispielsweise durch die Position der Quelle und des ausgelesenen Detektorpixels definiert.

Eine weitere Klasse der Rekonstruktionsmethoden sind die algebraischen Methoden, die durch numerische Lösung eines komplexen Gleichungssystems durch Optimierung eines Modells des untersuchten Objekts die Rekonstruktion erreicht. Diese Verfahren stellen keinerlei Voraussetzung an die geometrischen Rahmenbedingungen wie etwa abzudeckender Winkelbereich oder äuqidistante Abtastung der Projektionsbilder, sind jedoch äußerst rechenintensiv, prinzipiell jedoch im Kontext der Erfindung ebenso anwendbar.

Fig. 3 zeigt ein weiteres Ausführungsbeispiel einer Vorrichtung zum Erzeugen einer mehrdimensionalen Darstellung eines Messfelds 2, bei der die Röntgenquelle an mehrere Positionen bewegt werden kann, die in Fig. 3 schematisch dargestellt sind. Bei jeder Position kann das Messfeld 2 unter einer unterschiedlichen Perspektive aufgenommen werden, so dass eine mehrdimensionale Darstellung eines Messfelds 2 erzeugt werden kann, wenn die unter den unterschiedlichen Perspektiven aufgenommenen Aufnahmen kombiniert werden.

Bei dem in Fig. 3 gezeigten Ausführungsbeispiel werden sämtliche Bereiche des Messfelds mit unterschiedlichen Perspektiven aufgenommen, mit Ausnahme des tangential verlaufenden Zentralstrahls der Quelle. Aus mechanischen Gründen ist der Vergrößerung ein Limit gesetzt, das durch den Mindestabstand von der Röntgenquelle 4 zu dem Messfeld 2 gegeben ist, der durch Punkt 20 gegeben ist. Für Punkte bzw. Volumina innerhalb des Messfelds 2, die sich an der der Röntgenquelle abgewandten Seite des Messfelds 2 befinden, können Perspektivenänderungen von näherungsweise 90° realisiert werden, da bei einer entfernten Quelle der durch diese Volumina verlaufende Röntgenstrahl näherungsweise senkrecht zur Oberfläche des Detektors auftrifft, während dieselben Volumina aus einer näherungsweise parallel zur Detektoroberfläche verlaufenden Richtung beleuchtet werden, wenn sich die Röntgenquelle an Position 20 befindet.

Da eine Auflösung, mit der die Tiefeninformation, also die Koordinate in der Vorzugsrichtung 9 gewonnen werden kann, von dem Winkelbereich abhängt, unter dem einzelnen Volumina des Messfelds 2 betrachtet werden können, unterscheidet sich die erzielbare Ortsauflösung bzw. Tiefenauflösung je nach beobachtetem Teilvolumen des Messfelds 2. Da die Winkelvariation bei Bewegung der Röntgenquelle mit zunehmendem Abstand von der Achse, auf der die Röntgenquelle bewegt wird, zunimmt, nimmt die erzielbare Tiefenauflösung mit zunehmendem Abstand von der Röntgenquelle zu. Mit der in Fig. 2 gezeigten Vorrichtung können mithin durch die Translation einer einzelnen Röntgenquelle Daten innerhalb eines Winkelbereichs gemessen werden, der annähernd zwischen 0° und 90° liegt.

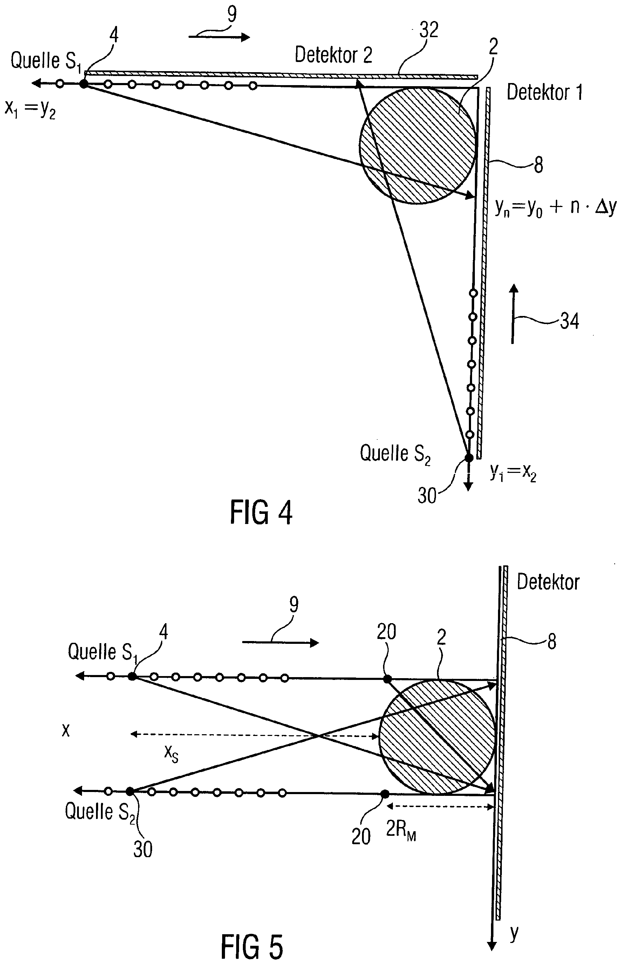

Fig. 4 zeigt ein weiteres Ausführungsbeispiel einer Vorrichtung zum Erzeugen einer mehrdimensionalen Darstellung, bei der zwei zueinander orthogonal ausgerichteten Quellen und ihnen jeweils zugeordnete Sensoren unabhängig voneinander bewegt werden können, so dass in Analogie zum oben beschriebenen Fall der Perspektivenbereich von 0 bis 90° verdoppelt werden kann. Somit können aus unterschiedlichen Perspektiven, die einen Winkel von annähernd 180° überspannen, Aufnahmen von dem Messfeld 2 gemacht werden. Dabei kann die Verschiebung der beiden Quellen simultan oder nacheinander erfolgen. Bei simultaner Aufnahme von Bildern ist damit zu rechnen, dass in den einzelnen Aufnahmen jeweils Streustrahlung der nicht den betreffenden Sensoren zugeordneten Röntgenquellen auftritt, was insgesamt den Kontrast in der Aufnahme verringern kann.

Mit anderen Worten zeigt das Ausführungsbeispiel in Fig. 4 eine zweite Röntgenquelle 30 zum Beleuchten des Messfelds 2. Die zweite Röntgenquelle 30 kann bezüglich einer zur Vorzugsrichtung 9 senkrechten zweiten Vorzugsrichtung 34 bewegt werden, welche durch die relative Position des Objekts bzw. des Messfeldes 2 und eines zweiten Sensors 32 gegeben ist. Die zweite Röntgenquelle 30 kann mittels einer zweiten Bewegungseinrichtung in der zweiten Vorzugsrichtung 34 bewegt werden, um einen Abstand in der zweiten Vorzugsrichtung zwischen der zweiten Röntgenquelle 30 und dem Messfeld 2 zu verändern. Äquivalent zum oben beschriebenen Verfahren kann mittels der zweiten Röntgenquelle 30 und des ihr zugeordneten zweiten Sensors 32 eine Folge von Aufnahmen des Messfelds 2 aufgenommen werden. Während der Datenauswertung mittels einer Kombinationseinrichtung wird die mehrdimensionale Darstellung dann unter Verwendung der Aufnahmen des ersten Sensors 8 und des zweiten Sensors 32 erhalten.

Durch die Verwendung der beiden orthogonal zueinander angeordneten Sensoren 8 und 32 mit den ihnen jeweils zugeordneten Röntgenquellen 34 kann, mittels Variation der Abstände der Röntgenquelle von dem zu untersuchenden Messfeld 2, erreicht werden, dass große Volumenanteile des zu untersuchenden Messfelds 2 näherungsweise unter einem Winkelbereich von 180° beobachtet werden können. Dies entspricht dem bei klassischer Rotations-CT verwendeten Winkelbereich, so dass die entsprechenden Bildanalysealgorithmen und Verfahren der CT-Rotations-Analyse zur Auswertung bzw. Kombination der mittels der Vorrichtung in Fig. 4 erzeugten Aufnahmen verwendet werden können.

Fig. 5 zeigt ein weiteres Ausführungsbeispiel, bei dem eine erste Röntgenquelle 4 und eine zweite Röntgenquelle 30 verwendet wird, wobei die erste und die zweite Röntgenquelle in einer Vorzugsrichtung, die durch die relative Orientierung des Messfelds 2 und des Sensors 8 gegeben ist, bewegt werden können, um das Messfeld 2 aus unterschiedlichen Perspektiven zu beleuchten, so dass, wenn beide Röntgenquellen 30 und 4 von den Punkten größten Abstands zum Messfeld 2 bis zu den Punkten geringsten Abstands 20 bewegt werden, Perspektiven aus einem Winkelbereich von annähernd 180° zur Verfügung stehen. Mit anderen Worten, werden zwei Röntgenquellen 30 und 4 verwendet, deren Verfahrwege parallel auf jeder Seite des Messfelds verlaufen. Dabei ergänzen sich die beiden Datensätze, die nacheinander aus gleichem Abstand jeder Quelle zum Messfeld aufgenommen werden, hinsichtlich Strahlwinkel und Entfernung zum Messfeldmittelpunkt spiegelsymmetrisch, so dass Daten aus einem 180°-Winkelbereich resultieren können.

Ähnlich wie das in Fig. 4 diskutierte Ausführungsbeispiel erlaubt das in Fig. 5 gezeigte Ausführungsbeispiel also, ein Messfeld aus unterschiedlichsten Perspektiven aufzunehmen, wobei das Messfeld bei dem in Fig. 5 gezeigten Ausführungsbeispiel insbesondere in einer Richtung parallel zum Sensor 8 stark ausgedehnt sein kann, ohne die Anwendung der erfindungsgemäßen Vorrichtung zu behindern.

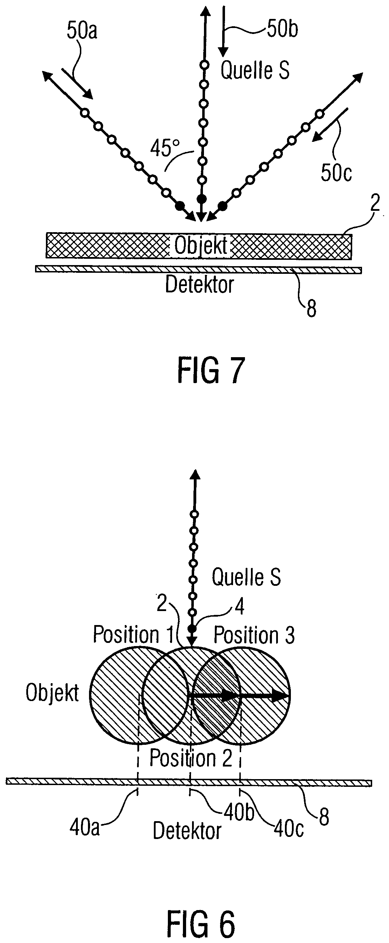

Insbesondere kann, wie in Fig. 6 gezeigt, bei einem ausgedehnten Messfeld das Messfeld relativ zum Sensor und zu der Röntgenquelle bzw. den Röntgenquellen 34 an der Vorrichtung vorbeibewegt werden (in einer lateralen Richtung), um das Messfeld 2 aus unterschiedlichen Perspektiven zu vermessen. So kann eine mehrdimensionale Darstellung des gesamten Messfelds erhalten werden, auch wenn dieses nicht vollständig in einem Messzyklus erfasst werden kann.

Fig. 6 zeigt am Beispiel der Verwendung einer einzelnen Röntgenquelle das Verfahren bzw. die Vorrichtung, die es erlaubt, das Messfeld 2 bzw. das Objekt in mehreren Iterationen an der Messvorrichtung vorbeizuführen. Dabei ist das Vorbeiführen bzw. das Verwenden einer zusätzlichen Translationseinrichtung, die das Objekt in einer Richtung senkrecht zur Vorzugsrichtung (in lateraler Richtung) bewegt, um dies beispielsweise an dem Sensor 8 bzw. der Röntgenquelle 4 vorbeizuführen, nicht auf dieses Ausführungsbeispiel beschränkt. Wie in Fig. 6 exemplarisch dargestellt, kann das Objekt oder Messfeld 2 in mehreren äquidistanten Positionen bzw. Schritten 40a, 40b und 40 c an Röntgenquelle 3 und Detektor 8 vorbeigeführt werden. Alternativ ist es selbstverständlich ebenfalls möglich, das Objekt nicht in äquidistanten Schritten zu bewegen, sofern es die geometrischen Randbedingungen erfordern. Auch muss die laterale Richtung bei keinem der Ausführungsbeispiele senkrecht zur Vorzugsrichtung sein. Diese kann einen beliebigen Winkel mit der Vorzugsrichtung einschließen, beispielsweise einen oder mehrere Winkel zwischen 20° und 70°.

Mit anderen Worten ist eine Translationseinrichtung, wie oben beschrieben, bei allen der übrigen Ausführungsbeispiele einsetzbar, bei denen eine oder mehrere Strahlungsquellen bezüglich des Objekts bei feststehendem Detektor bewegt werden.

Ferner kann eine Translationseinrichtung dazu verwendet werden, die Perspektive, unter der das Objekt an den verschiedenen Positionen der Translationsbewegung aufgenommen wird, in großen Winkelschritten zu variieren, wohingegen kleine Perspektivenänderungen durch die Bewegung der Röntgenquelle in der Vorzugsrichtung vorgenommen werden. Durch die Kombination von großen Winkelinkrementen mit kleinen Winkelinkrementen kann die Auflösung noch weiter verbessert werden.

Fig. 7 zeigt ein weiteres Ausführungsbeispiel, mittels dessen, basierend auf dieser Idee die Auflösung weiter verbessert werden kann.

Dazu ist in Fig. 7 schematisch eine Rotationseinrichtung dargestellt, mittels derer die Vorzugsrichtung, in der die Röntgenquelle relativ zum Objekt bewegt wird, variiert werden kann. Bei Verwendung einer derartigen Vorrichtung bzw. bei Anwenden eines dieser Vorrichtung entsprechenden Verfahrens, kann ein Messfeld 2 unter Verwendung mehrerer Vorzugsrichtungen mittels eines Sensors 8 untersucht werden. Fig. 7 zeigt ein Beispiel mit drei Vorzugsrichtungen 50a, 50b und 50c, die sich jeweils durch ein Winkelinkrement von 45° voneinander unterscheiden. Große Perspektivenänderungen können somit durch die Verkippung der Vorzugsrichtungen bzw. der die Quelle führenden Bewegungseinrichtung verursacht werden. Die kleineren Perspektivenänderungen, die auf der Bewegung der Röntgenquelle 4 der Vorzugsrichtung beruhen, liefern zusätzlich die zusätzlichen Perspektiven in kleinen Winkelinkrementen, die zum Erreichen einer hohen Ortsauflösung erforderlich sind.

Alternativ zur Rotation bzw. Verkippung der Bewegungseinrichtung kann, mit dem gleichen Effekt, das Messfeld 2 mit dem Objekt selbst um die entsprechenden Winkelinkremente verkippt bzw. rotiert werden. Mit anderen Worten kann ein anhand von Fig. 7 schematisch beschriebenes Ausführungsbeispiel alternativ oder ergänzend zu den Verfahren mit mehreren Detektor-Quellen-Paaren verwendet werden, bei dem eine oder wenige Drehbewegungen des Objekts oder der Detektorachse die Vollständigkeit der für die CT-Rekonstruktion benötigten Daten stark verbessern, wobei mit vergleichsweise geringer Präzision um den erforderlichen großen Winkel (beispielsweise den hier gezeigten 45° Winkel) rotiert werden muss. Alternativ können selbstverständlich auch andere Winkelinkremente, wie beispielsweise 22,5° verwendet werden. Auch hier kann alternativ das Objekt selbst relativ zur Strahlungsquelle ein oder wenige Male um den entsprechenden Winkel verkippt werden, wenn es die geometrischen Randbedingungen erlauben.

Somit kann also mittels des erfindungsgemäßen Konzepts durch Rotation um wenige große Winkelinkremente eine vollständige Rekonstruktion eines zu untersuchenden Objekts im Messfeld erreicht werden. Dabei beträgt die Anzahl der Projektionsrichtungen, also der Rotationen, bevorzugt weniger als 10 und bei besonders bevorzugten Ausführungsbeispielen weniger als 5. Die Winkelinkremente der Rotation können dabei bei bevorzugten Ausführungsbeispielen größer oder gleich 10° sein, also wesentlich größer als bei herkömmlichen rotations-basierten CT-Verfahren. Auch kann der maximale Winkelbereich, unter dem das Objekt betrachtet wird, kleiner oder gleich 90° sein, d.h. die maximale Rotation kann geringer als 90° sein, wobei trotzdem eine vollständige Rekonstruktion innerhalb der ROI erreicht werden kann.

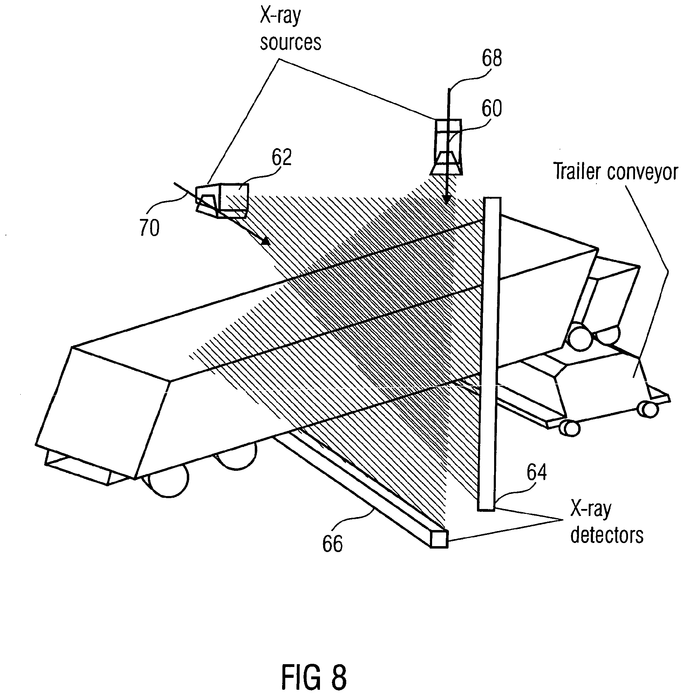

Fig. 8 zeigt schematisch eine mögliche Anwendung des Verfahrens bzw. erfindungsgemäßer Vorrichtungen, um Frachtcontainer zu untersuchen bzw. dreidimensionale Schnittbilder bzw. Darstellungen von Frachtcontainern bei einer mobilen oder ortsfesten Kontrolle zu erzeugen. Dabei kann ein möglicherweise bereits bestehendes System aus einer ersten Röntgenquelle 60 und einer zweiten Röntgenquelle 62 und ihnen jeweils auf gegenüberliegenden Seiten des zu untersuchenden Objekts zugeordneten Röntgendetektoren 64 und 66 erweitert werden, indem eine Bewegungsvorrichtung implementiert wird, die es erlaubt, die Röntgenquelle 60 in der ersten Vorzugsrichtung 68 und die zweite Röntgenquelle 62 in der zweiten Vorzugsrichtung 70 relativ zum Container zu bewegen. Bei Anwendung des oben beschriebenen Konzepts kann eine dreidimensionale Darstellung des Inhalts des Containers erzeugt werden kann.

Somit kann beispielsweise ein System erweitert werden, bei dem bereits mittels zweier orthogonaler Quellen (beispielsweise zwei LINACS, die im Winkel von 90° zueinander angeordnet sind) und zugehörigen Zeilen- bzw. Flächendetektoren ein Objekt schichtweise gescannt wird, indem die zu untersuchenden Bereiche an den Detektoren vorbei bewegt werden. Alternativ kann auch das Detektorsystem an den zu untersuchenden Bereichen vorbei bewegt werden. In dem anhand von Fig. 8 beschriebenen Anwendungsszenario ist ein besonders evidenter Vorteil, dass eine Rotation der Detektorkomponenten bzw. des Objekts vermieden werden kann, die bei den beschriebenen Containern nur mit einem sehr hohen Aufwand realisierbar wäre, der bei Anwendung des oben beschriebenen Konzepts entfallen kann.

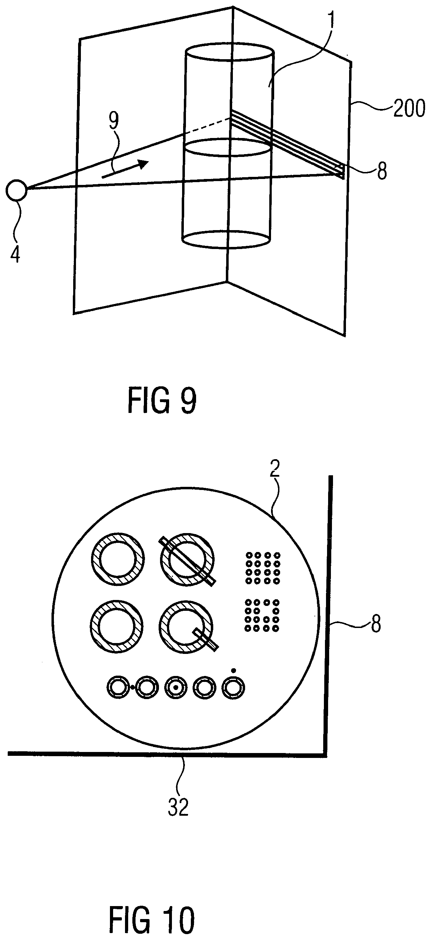

Fig. 9 und 10 zeigen Beispiele für die Anwendung des erfindungsgemäßen Konzepts zur Vermessung ortsfester Objekte, die aufgrund ihrer Einbausituation nicht rotiert oder bewegt werden können. Dabei basieren die in Fig. 9 und 10 gezeigten Ausführungsbeispiele auf einer räumlichen Anordnung von Röntgenquellen und Detektoren, wie sie beispielsweise anhand von Fig. 4 bereits schematisch dargestellt wurde. Bezüglich der Details der Auswertung sei daher an dieser Stelle auf die Ausführungen zu den Fig. 3 und 4 verwiesen.

Fig. 9 zeigt als zu vermessendes Objekt 1 ein Rohr, welches in einer für eine normale Röntgenuntersuchung ungünstigen Einbaulage in der Ecke einer Wand 200 installiert ist. Wie aus der Figur ersichtlich, verhindert die Wand 200 eine Rotationsbewegung der Röntgenquelle 4 und des Detektors 8 um das Rohr. Bei Anwendung des erfindungsgemäßen Konzepts kann das Rohr jedoch trotzdem mehrdimensional vermessen werden, indem die Röntgenquelle 4 in der Vorzugsrichtung 9 bewegt wird, wie es anhand der Prinzipskizzen in den Fig. 4 und 5 bereits ausführlich erläutert wurde.

Fig. 10 zeigt eine weitere Möglichkeit der industriellen Anwendung, beispielsweise in einer Raffinerie, wo eine Vielzahl von innerhalb des Messfeldes 2 befindlichen Rohren bzw. Kabeln unterschiedlichen Querschnitts simultan untersucht werden können, wenn beispielsweise die schematisch skizzierte Anordnung mit zwei Sensoren 8 und 32 verwendet wird, die rechtwinklig zueinander angeordnet sind, wie es u.a. auch anhand von Fig. 4 diskutiert wurde.

Zusammenfassend kann ein Verfahren zur Erzeugung einer mehrdimensionalen Darstellung eines Objekts, dass das in dieser Anmeldung beschriebene Konzept umsetzt, wie folgt beschrieben werden.

Zunächst wird eine erste und eine zweite Aufnahme des mit einer Röntgenquelle beleuchteten Objekts mit einem Sensor erzeugt, der sich bezüglich der Röntgenquelle in eine durch die relative Position des Objekts und des Sensors gegebenen Vorzugsrichtung hinter dem Objekt befindet, wobei ein Abstand in der Vorzugsrichtung zwischen der Röntgenquelle und dem Objekt sich zwischen der ersten und der zweiten Aufnahme unterscheidet. Mit anderen Worten wird also zwischen der ersten und der zweiten Aufnahme dieser Abstand verändert. Folglich wird eine erste Aufnahme mit einem ersten Abstand zwischen der Röntgenquelle und dem Objekt durchgeführt, woraufhin eine zweite Aufnahme mit einem zweiten Abstand zwischen der Röntgenquelle und dem Objekt durchgeführt wird.

Um die mehrdimensionale Darstellung des Objekts zu erhalten, wird dann die erste und die zweite Aufnahme kombiniert, wobei an die geometrischen Bedingungen angepasste Bildrekonstruktionsalgorithmen für Röntgentomographie verwendet werden können.

Obwohl bei den oben beschriebenen Ausführungsbeispielen maximal jeweils zwei unterschiedliche Röntgenquellen verwendet wurden, ist das beschriebene Konzept selbstverständlich auch auf eine größere Anzahl von Röntgenquellen anwendbar, wobei sich insbesondere beispielsweise die Notwendigkeit der Rotation des Objekts bzw. der Achsen der Vorzugsrichtung erübrigt, wenn mehrere Quellen verwendet werden.

Wie bereits erwähnt, ist es alternativ zum Verändern der Position der Röntgenquelle ebenfalls möglich, die Position des Objekts selbst in Richtung der Röntgenquelle zu verändern, um die erforderlichen unterschiedlichen Perspektiven der Abbildung zu erhalten. Ferner ist es möglich, bei feststehendem Objekt den Detektor zu verfahren, um die Vergrößerung der Abbildung stufenweise zu ändern und so ggf. einzelne Bereiche des zu untersuchenden Objekts mit höherer Auflösung abzubilden. Diese Möglichkeit ist selbstverständlich als zusätzliche Eigenschaft mit allen im Vorhergehenden diskutierten Ausführungsbeispielen kombinierbar.

In den oben beschriebenen Ausführungsbeispielen wurde aufgrund der Einfachheit der Darstellung eine jeweils konstante Schrittweite bei Bewegung der Röntgenquelle bzw. des Objekts gezeigt. Daraus ergibt sich jedoch eine jeweils unterschiedliche Änderung des Winkels und somit der Perspektive. Um, ähnlich wie in Standard CT-Algorithmen, eine äquidistante Winkeländerung je Aufnahme zu erhalten, ist es ferner möglich, die Translationsbewegung der Quelle so durchzuführen, dass sich daraus eine äquidistante Winkeländerung, also eine jeweils konstante Perspektivenänderung ergibt.

Bei einigen weiteren Ausführungsbeispielen können die Translationsbewegungen beispielsweise auch mit Roboterarmen durchgeführt werden, was eine erhöhte Flexibilität des Verfahrens zur Folge hat, indem die Quellen auch bei äußerst unregelmäßig geformten zu untersuchenden Objekten angewendet werden kann, indem in allen räumlichen Freiheitsgraden Bewegungen der Röntgenquelle durchgeführt werden können.

Zusammenfassend ergeben sich aus der Anwendung der oben beschriebenen Vorrichtungen bzw. Verfahren zahlreiche Vorteile. Beispielsweise kann eine mechanisch schwierig zu realisierende Vorrichtung zum Durchführen hochpräziser Drehbewegungen vermieden werden. Dadurch können u.a. auch Auflösungsverluste durch Achsentaumeln bzw. Fehljustage solcher komplexen mechanischen Vorrichtungen vermieden werden.

Auch können nunmehr Objekte mit ungünstiger Geometrie (beispielsweise Flugzeugflügel oder Leiterplatten) unter fast vollständigem Verzicht einer Objektbewegung einer vollständigen dreidimensionalen Computertomographie unterzogen werden.

Abhängig von den Gegebenheiten kann das Verfahren zur Erzeugung einer mehrdimensionalen Darstellung eines Objekts in Hardware oder in Software implementiert werden. Die Implementation kann auf einem digitalen Speichermedium, insbesondere einer Diskette oder CD mit elektronisch auslesbaren Steuersignalen erfolgen, die so mit einem programmierbaren Computersystem zusammenwirken können, dass das Verfahren zur Erzeugung einer mehrdimensionalen Darstellung eines Objekts ausgeführt wird. Allgemein besteht die Erfindung somit auch in einem Computer-Programm-Produkt mit einem auf einem maschinenlesbaren Träger gespeicherten Programmcode zur Durchführung des erfindungsgemäßen Verfahrens, wenn das Computer-Programm-Produkt auf einem Rechner abläuft. In anderen Worten ausgedrückt kann die Erfindung somit als ein Computer-Programm mit einem Programmcode zur Durchführung des Verfahrens realisiert werden, wenn das Computer-Programm auf einem Computer abläuft.

## Patentansprüche

1. Vorrichtung zum Erzeugen einer mehrdimensionalen Darstellung eines Objekts (2), mit folgenden Merkmalen:
einer Röntgenquelle (4; 60) zum Beleuchten des Objekts (2) und einer weiteren Röntgenquelle (30; 62) zum Beleuchten des Objekts (2);
einem Sensor (8; 66), der sich bezüglich der Röntgenquelle (4; 60) in der durch die relative Position des Objekts (2) und des Sensors (8; 66) gegebenen Vorzugsrichtung (9; 68) hinter dem Objekt (2) befindet, zum Erzeugen einer ersten Aufnahme und einer zweiten Aufnahme des Objekts (2),
wobei sich die weitere Röntgenquelle (30; 62) in einer weiteren Vorzugsrichtung (34; 70) vor dem Objekt (2) befindet;
einer Bewegungseinrichtung zum Bewegen der Röntgenquelle (4; 60) oder des Objekts (2), um einen Abstand in der Vorzugsrichtung (9; 68) zwischen der Röntgenquelle (4; 60) und dem Objekt (2) zwischen der ersten und der zweiten Aufnahme zu verändern, wobei die Bewegungseinrichtung zusätzlich ausgebildet ist, um die weitere Röntgenquelle (30; 62) in der weiteren Vorzugsrichtung zu bewegen, um einen weiteren Abstand in der weiteren Vorzugsrichtung (34; 70) zwischen der weiteren Röntgenquelle (30; 62) und dem Objekt (2) zu verändern; und
einer Kombinationseinrichtung (12) die ausgebildet ist, um aus der Veränderung des Abstands zwischen der ersten und der zweiten Aufnahme einen Rotationswinkel, der eine Rotation der Röntgenquelle (4; 60) bezüglich des Objekts (2) beschreibt, zu bestimmen, welcher zu einer Veränderung der Perspektive, unter der das Objekt (2) beleuchtet und auf dem Sensor (8; 66) abgebildet wird, korrespondiert, und
wobei die Kombinationseinrichtung (12) ferner ausgebildet ist, um die erste Aufnahme und die zweite Aufnahme unter Verwendung des Rotationswinkels und eines Computer-Tomographie-Bildverarbeitungsalgorithmus zu kombinieren, um die mehrdimensionale Darstellung des Objekts (2) zu erhalten,
wobei der Sensor (8) zusätzlich ausgebildet ist, um eine dritte Aufnahme zu erzeugen, bei der das Objekt (2) von der weiteren Röntgenquelle (30) beleuchtet wird, wobei die Kombinationseinrichtung (12) ausgebildet ist, um die erste Aufnahme, die zweite Aufnahme und die dritte Aufnahme zu kombinieren, um die mehrdimensionale Darstellung des Objekts (2) zu erhalten.

2. Vorrichtung zum Erzeugen einer mehrdimensionalen Darstellung eines Objekts (2), mit folgenden Merkmalen:
einer Röntgenquelle (4; 60) zum Beleuchten des Objekts (2) und einer weiteren Röntgenquelle (30; 62) zum Beleuchten des Objekts (2);
einem Sensor (8; 66), der sich bezüglich der Röntgenquelle (4; 60) in der durch die relative Position des Objekts (2) und des Sensors (8; 66) gegebenen Vorzugsrichtung (9; 68) hinter dem Objekt (2) befindet, zum Erzeugen einer ersten Aufnahme und einer zweiten Aufnahme des Objekts (2),
wobei sich die weitere Röntgenquelle (30; 62) in einer weiteren Vorzugsrichtung (34; 70) vor dem Objekt (2) befindet;
einer Bewegungseinrichtung zum Bewegen der Röntgenquelle (4; 60) oder des Objekts (2), um einen Abstand in der Vorzugsrichtung (9; 68) zwischen der Röntgenquelle (4; 60) und dem Objekt (2) zwischen der ersten und der zweiten Aufnahme zu verändern, wobei die Bewegungseinrichtung zusätzlich ausgebildet ist, um die weitere Röntgenquelle (30; 62) oder das Objekt (2) in der weiteren Vorzugsrichtung zu bewegen, um einen weiteren Abstand in der weiteren Vorzugsrichtung (34; 70) zwischen der weiteren Röntgenquelle (30; 62) und dem Objekt (2) zu verändern; und
einer Kombinationseinrichtung (12) die ausgebildet ist, um aus der Veränderung des Abstands zwischen der ersten und der zweiten Aufnahme einen Rotationswinkel, der eine Rotation der Röntgenquelle (4; 60) bezüglich des Objekts (2) beschreibt, zu bestimmen, welcher zu einer Veränderung der Perspektive, unter der das Objekt (2) beleuchtet und auf dem Sensor (8; 66) abgebildet wird, korrespondiert, und
wobei die Kombinationseinrichtung (12) ferner ausgebildet ist, um die erste Aufnahme und die zweite Aufnahme unter Verwendung des Rotationswinkels und eines Computer-Tomographie-Bildverarbeitungsalgorithmus zu kombinieren, um die mehrdimensionale Darstellung des Objekts (2) zu erhalten,
wobei die Vorzugsrichtung (9, 68) senkrecht zu der weiteren Vorzugsrichtung (34, 70) ist, wobei die weitere Vorzugsrichtung (34; 70) durch die relative Position des Objekts (2) und eines weiteren Sensors (32; 64) gegeben ist, und wobei die weitere Röntgenquelle (30, 70) vor dem Objekt (2) angeordnet ist, wobei die Vorrichtung ferner folgendes Merkmal aufweist:
den weiteren Sensor (32, 64), der ausgebildet ist, um eine dritte Aufnahme zu erzeugen, bei der das Objekt (2) von der weiteren Röntgenquelle (30; 70) beleuchtet wird, und
wobei die Kombinationseinrichtung (12) ausgebildet ist, um die erste Aufnahme, die zweite Aufnahme, und die dritte Aufnahme zu kombinieren, um die mehrdimensionale Darstellung des Objekts (2) zu erhalten.

3. Vorrichtung gemäß Anspruch 1 oder 2, bei der die Vorzugsrichtung (9) senkrecht zu einer sensitiven Oberfläche des Sensors (8) ist.

4. Vorrichtung gemäß einem der Ansprüche 1 bis 3, bei der eine mehrdimensionale Darstellung erzeugt wird, die Informationen über eine zusätzliche, weder in der ersten noch in der zweiten Aufnahme enthaltenen, räumlichen Dimension, enthält, sodass in der mehrdimensionalen Darstellung mindestens eine räumliche Dimension mehr als in der ersten Aufnahme und der zweiten Aufnahme abgebildet wird.

5. Vorrichtung gemäß einem der Ansprüche 1 bis 4, bei der die Kombinationseinrichtung (12) ausgebildet ist, um eine durch die Veränderung des Abstands in der Vorzugsrichtung (9) zwischen den Aufnahmen hervorgerufene Maßstabsänderung in der ersten Aufnahme oder der zweiten Aufnahme zu kompensieren.

6. Vorrichtung gemäß Anspruch 5, bei der die Kombinationseinrichtung (12) ausgebildet ist, um die Maßstabsänderung durch einen von dem Abstand in der Vorzugsrichtung (9) abhängigen Skalierungsfaktor zu kompensieren.

7. Vorrichtung gemäß einem der vorhergehenden Ansprüche, mit folgendem zusätzlichen Merkmal:
einer Rotationseinrichtung, um die Vorzugsrichtung (50a, 50b, 50c) bezüglich des Objekts (2) um einen vorbestimmten Winkel zu rotieren.

8. Vorrichtung gemäß einem der vorhergehenden Ansprüche, mit folgendem zusätzlichen Merkmal:
einer Translationseinrichtung, um in einer lateralen Bewegungsrichtung, die mit der Vorzugsrichtung (9) einen vorbestimmten Winkel einschließt, eine Relativbewegung (40a, 40b, 40c) von Objekt (2) und Röntgenquelle (4) durchzuführen.

9. Vorrichtung gemäß Anspruch 8, bei der die Translationseinrichtung ausgebildet ist, um die Röntgenquelle (4) in einer Richtung senkrecht zu der Vorzugsrichtung (9) zu bewegen.

10. Vorrichtung gemäß Anspruch 9, bei der die Translationseinrichtung ausgebildet ist, um das Objekt (2) in einer Richtung senkrecht zu der Vorzugsrichtung zu bewegen.

11. Vorrichtung gemäß Anspruch 1,
bei der die Röntgenquelle (60) und die weitere Röntgenquelle (62) in einem Winkel von 90° zueinander angeordnet sind,
bei der ferner zusätzlich zu dem Sensor (66) ein weiterer Sensor (64) vorhanden ist,
bei der der Sensor (66) auf einer bezüglich des Objekts (2) gegenüberliegenden Seite der Röntgenquelle (60) angeordnet ist,
bei der der weitere Sensor (64) auf einer bezüglich des Objekts (2) gegenüberliegenden Seite der weiteren Röntgenquelle (60) angeordnet ist,
bei der die Vorzugsrichtung (68) senkrecht zu der weiteren Vorzugsrichtung (70) ist; und
bei der die Bewegungseinrichtung eine gemeinsame Bewegungseinrichtung ist, um das Objekt (2) in einer auf der Vorzugsrichtung (68) und der weiteren Vorzugsrichtung (70) senkrecht stehenden Bewegungsrichtung relativ zu der Röntgenquelle (60) und dem Sensor (66)und relativ zu der weiteren Röntgenquelle (62) und dem weiteren Sensor (64)zu bewegen, oder um alternativ ein Detektorsystem, das die Röntgenquelle (60) und den Sensor (66) und die weitere Röntgenquelle (62) und den weiteren Sensor (64) aufweist, an zu untersuchenden Bereichen des Objekts (2) vorbei zu bewegen.

12. Vorrichtung gemäß Anspruch 1,
bei der die Vorzugsrichtung (9) und die weitere Vorzugsrichtung (34, 70) gleich sind, und durch eine relative Orientierung des Objekts (2) und des Sensors (8) gegeben sind, und
bei der die Röntgenquelle (4) und die weitere Röntgenquelle (30) verwendet werden, deren Verfahrwege parallel auf jeder Seite des Objekts (2) verlaufen, und
bei der die Röntgenquelle (4) und die weitere Röntgenquelle (30) von Punkten größten Abstands zu dem Objekt (2) bis zu Punkten (20) geringsten Abstands dem Objekt (2) bewegt werden, um Perspektiven aus einem Winkelbereich von annähernd 180° zu erhalten.

13. Computer-implementiertes Verfahren zum Erzeugen einer mehrdimensionalen Darstellung eines Objekts (2), umfassend:
Erzeugen einer ersten und einer zweiten Aufnahme des mit einer Röntgenquelle (4; 60) beleuchteten Objekts (2) mit einem Sensor (8; 66), der sich bezüglich der Röntgenquelle (4; 60) in einer durch eine relative Position des Objekts (2) und des Sensors (8; 66) gegebenen Vorzugsrichtung (9; 68) hinter dem Objekt (2) befindet,
wobei ein Abstand in der Vorzugsrichtung (9; 68) zwischen der Röntgenquelle (4; 60) und dem Objekt (2) sich zwischen der ersten Aufnahme und der zweiten Aufnahme unterscheidet,
wobei eine weitere Röntgenquelle (30; 62) zum Beleuchten des Objekts (2) in einer weiteren Vorzugsrichtung (34; 70) vor dem Objekt (2) vorgesehen ist,
wobei die weitere Röntgenquelle (30; 62) in der weiteren Vorzugsrichtung (34; 70) bewegt wird, um einen weiteren Abstand in der weiteren Vorzugsrichtung (34; 70) zwischen der weiteren Röntgenquelle (30, 62) und dem Objekt (2) zu verändern; und
Bestimmen eines Rotationswinkels aus der Veränderung des Abstands zwischen der ersten Aufnahme und der zweiten Aufnahme, der eine Rotation der Röntgenquelle (4; 60) bezüglich des Objekts (2) beschreibt, die zu einer Veränderung der Perspektive, unter der das Objekt (2) beleuchtet und auf dem Sensor (8; 66) abgebildet wird, korrespondiert; und
Kombinieren der ersten Aufnahme und der zweiten Aufnahme unter Verwendung des Rotationswinkels und eines Computer-Tomographie-Bildverarbeitungsalgorithmus, um die mehrdimensionale Darstellung des Objekts (2) zu erhalten,
Erzeugen einer dritten Aufnahme mit dem Sensor (8), wobei das Objekt (2) von der weiteren Röntgenquelle (30) beleuchtet wird, wobei das Kombinieren das Kombinieren der ersten Aufnahme, der zweiten Aufnahme und der dritten Aufnahme aufweist, um die mehrdimensionale Darstellung des Objekts (2) zu erhalten.

14. Computer-implementiertes Verfahren zum Erzeugen einer mehrdimensionalen Darstellung eines Objekts (2), umfassend:
Erzeugen einer ersten und einer zweiten Aufnahme des mit einer Röntgenquelle (4; 60) beleuchteten Objekts (2) mit einem Sensor (8; 66), der sich bezüglich der Röntgenquelle (4; 60) in einer durch eine relative Position des Objekts (2) und des Sensors (8; 66) gegebenen Vorzugsrichtung (9; 68) hinter dem Objekt (2) befindet,
wobei ein Abstand in der Vorzugsrichtung (9; 68) zwischen der Röntgenquelle (4; 60) und dem Objekt (2) sich zwischen der ersten Aufnahme und der zweiten Aufnahme unterscheidet,
wobei eine weitere Röntgenquelle (30; 62) zum Beleuchten des Objekts (2) in einer weiteren Vorzugsrichtung (34; 70) vor dem Objekt (2) vorgesehen ist,
wobei die weitere Röntgenquelle (30; 62) oder das Objekt (2) in der weiteren Vorzugsrichtung (34; 70) bewegt wird, um einen weiteren Abstand in der weiteren Vorzugsrichtung (34; 70) zwischen der weiteren Röntgenquelle (30, 62) und dem Objekt (2) zu verändern; und
Bestimmen eines Rotationswinkels aus der Veränderung des Abstands zwischen der ersten Aufnahme und der zweiten Aufnahme, der eine Rotation der Röntgenquelle (4; 60) bezüglich des Objekts (2) beschreibt, die zu einer Veränderung der Perspektive, unter der das Objekt (2) beleuchtet und auf dem Sensor (8; 66) abgebildet wird, korrespondiert; und
Kombinieren der ersten Aufnahme und der zweiten Aufnahme unter Verwendung des Rotationswinkels und eines Computer-Tomographie-Bildverarbeitungsalgorithmus, um die mehrdimensionale Darstellung des Objekts (2) zu erhalten,
wobei die Vorzugsrichtung (9, 68) senkrecht zu der weiteren Vorzugsrichtung (34, 70) ist, wobei die weitere Vorzugsrichtung (34; 70) durch die relative Position des Objekts (2) und eines weiteren Sensors (32; 64) gegeben ist, und wobei die weitere Röntgenquelle (30, 70) vor dem Objekt (2) angeordnet ist, wobei das Verfahren ferner aufweist: Erzeugen einer dritten Aufnahme durch den weiteren Sensor (32, 64), wobei das Objekt (2) von der weiteren Röntgenquelle (30; 70) beleuchtet wird, wobei das Kombinieren das Kombinieren der ersten Aufnahme, der zweiten Aufnahme, und der dritten Aufnahme umfasst, um die mehrdimensionale Darstellung des Objekts (2) zu erhalten.

15. Computer-Programm mit einem Programmcode zur Ausführung des Verfahrens von Anspruch 13 oder 14, wenn das Programm auf einem Computer abläuft.

## Claims

1. An apparatus for creating a multi-dimensional representation of an object (2), comprising:
an X-ray source (4; 60) for illuminating the object (2) and a further X-ray source (30; 62) for illuminating the object (2);
a sensor (8; 66) located, in relation to the X-ray source (4; 60), behind the object (2) in the preferential direction (9; 68) defined by the relative positions of the object (2) and of the sensor (8; 66), for creating a first picture and a second picture of the object (2);
the further X-ray source (30; 62) being located in front of the object (2) in a further preferential direction (34; 70);
a mover for moving the X-ray source (4; 60) or the object (2) so as to change a distance in the preferential direction (9; 68) between the X-ray source (4; 60) and the object (2) between the first and second pictures, the mover additionally being configured to move the further X-ray source (30; 62) in the further preferential direction so as to change a further distance in the further preferential direction (34; 70) between the further X-ray source (30; 62) and the object (2); and
a combiner (12) configured to determine, from the change in the distance between the first and second pictures, an angle of rotation which describes a rotation of the X-ray source (4; 60) in relation to the object (2) which corresponds to a change in the perspective from which the object (2) is illuminated and imaged on the sensor (8; 66), and
the combiner (12) further being configured to combine the first and second pictures while using the angle of rotation and a computer tomography image processing algorithm in order to acquire the multi-dimensional representation of the object (2), the sensor (8) additionally being configured to create a third picture in which the object (2) is illuminated by the further X-ray source (30), the combiner (12) being configured to combine the first picture, the second picture and the third picture so as to acquire the multi-dimensional representation of the object (2).

2. An apparatus for creating a multi-dimensional representation of an object (2), comprising:
an X-ray source (4; 60) for illuminating the object (2) and a further X-ray source (30; 62) for illuminating the object (2);
a sensor (8; 66) located, in relation to the X-ray source (4; 60), behind the object (2) in the preferential direction (9; 68) defined by the relative positions of the object (2) and of the sensor (8; 66), for creating a first picture and a second picture of the object (2);
the further X-ray source (30; 62) being located in front of the object (2) in a further preferential direction (34; 70);
a mover for moving the X-ray source (4; 60) or the object (2) so as to change a distance in the preferential direction (9; 68) between the X-ray source (4; 60) and the object (2) between the first and second pictures, the mover additionally being configured to move the further X-ray source (30; 62) or the object (2) in the further preferential direction so as to change a further distance in the further preferential direction (34; 70) between the further X-ray source (30; 62) and the object (2); and
a combiner (12) configured to determine, from the change in the distance between the first and second pictures, an angle of rotation which describes a rotation of the X-ray source (4; 60) in relation to the object (2) which corresponds to a change in the perspective from which the object (2) is illuminated and imaged on the sensor (8; 66), and
the combiner (12) further being configured to combine the first and second pictures while using the angle of rotation and a computer tomography image processing algorithm in order to acquire the multi-dimensional representation of the object (2), the preferential direction (9, 68) being perpendicular to the further preferential direction (34, 70), wherein the further preferential direction (34; 70) is defined by the relative positions of the object (2) and of the further sensors (32; 64), and wherein the further X-ray source (30, 70) is arranged in front of the object (2), the apparatus further comprising:
the further sensor (32, 64) being configured to create a third picture in which the object (2) is illuminated by the further X-ray source (30; 70), and
the combiner (12) further being configured to combine the first picture, the second picture and the third picture in order to acquire the multi-dimensional representation of the object (2).

3. The apparatus as claimed in claim 1 or 2, wherein the preferential direction (9) is perpendicular to a sensitive surface of the sensor (8).

4. The apparatus as claimed in any of claims 1 to 3, wherein a multi-dimensional representation is created which comprises information about an additional spatial dimension comprised neither by the first nor by the second picture, so that at least one more spatial dimension is imaged in the multi-dimensional representation than in the first picture and the second picture.

5. The apparatus as claimed in any of claims 1 to 4, wherein the combiner (12) is configured to compensate for a change in scale within the first picture or second picture which is due to the change in the distance in the preferential direction (9) between the pictures.

6. The apparatus as claimed in claim 5, wherein the combiner (12) is configured to compensate for a change in scale by a scaling factor dependent on the distance in the preferential direction (9).

7. The apparatus as claimed in any of the preceding claims, further comprising:
a rotator for rotating the preferential direction (50a, 50b, 50c) by a predetermined angle with regard to the object (2).

8. The apparatus as claimed in in any of the preceding claims, further comprising:
a translator for performing a relative motion (40a, 40b, 40c) of the object (2) and the X-ray source (4) in a lateral direction of motion that forms a predetermined angle with the preferential direction (9).

9. The apparatus as claimed in claim 8, wherein the translator is configured to move the X-ray source (4) in a direction perpendicular to the preferential direction (9).

10. The apparatus as claimed in claim 9, wherein the translator is configured to move the object (2) in a direction perpendicular to the preferential direction.

11. The apparatus as claimed in claim 1,
wherein the X-ray source (60) and the further X-ray source (62) are arranged at an angle of 90° relative to each other,
wherein, additionally to the sensor (66), a further sensor (64) is further provided,
wherein the sensor (66) is arranged opposite the X-ray source (60) in relation to the object (2),
wherein the further sensor (64) is arranged opposite the further X-ray source (60) in relation to the object (2),
wherein the preferential direction (68) is perpendicular to the further preferential direction (70); and
wherein the mover is a common mover so as to move the object (2) in a direction of motion which is perpendicular to the preferential direction (68) and the further preferential direction (70) in relation to the X-ray source (60) and the sensor (66) and in relation to the further X-ray source (62) and the further sensor (64), or, alternatively, so as to move a detector system which comprises the X-ray source (60) and the sensor (66) and the further X-ray source (62) and the further sensor (64) past the regions to be examined of the object (2).

12. The apparatus as claimed in claim 1,
wherein the preferential direction (9) and the further preferential direction (34, 70) are the same and are defined by a relative orientation of the object (2) and of the sensor (8), and
wherein use is made of the X-ray source (4) and the further X-ray source (30), the travel paths of which are parallel on each side of the object (2), and
wherein the X-ray source (4) and the further X-ray source (30) are moved from points of the largest distance to the object (2) to points (20) of the smallest distance to the object (2) in order to acquire perspectives from a range of angles of approximately 180°.

13. Computer-implemented method for creating a multi-dimensional representation of an object (2), including:
creating a first and a second picture of the object (2) illuminated by the X-ray source (4; 60) using a sensor (8; 66) which is located, in relation to the X-ray source (4; 60), behind the object (2) in a preferential direction (9; 68) defined by a relative position of the object (2) and of the sensor (8; 66),
wherein a distance in the preferential direction (9; 68) between the X-ray source (4; 60) and the object (2) is different in the first picture and in the second picture,
wherein a further X-ray source (30; 62) for illuminating the object (2) is provided in front of the object (2) in a further preferential direction (34; 70),
wherein the further X-ray source (30; 62) is moved in the further preferential direction (34; 70) so as to change a further distance in the further preferential direction (34; 70) between the further X-ray source (30, 62) and the object (2); and
determining, from the change in the distance between the first and second pictures, an angle of rotation which describes a rotation of the X-ray source (4; 60) in relation to the object (2) which corresponds to a change in the perspective from which the object (2) is illuminated and imaged on the sensor (8; 66); and
combining the first and second pictures while using the angle of rotation and a computer tomography image processing algorithm in order to acquire the multi-dimensional representation of the object (2),
creating a third picture while using the sensor (8), wherein the object (2) is illuminated by the further X-ray source (30), wherein combining comprises combining of the first picture, the second picture and the third picture in order to acquire the multi-dimensional representation of the object (2).

14. Computer-implemented method for creating a multi-dimensional representation of an object (2), including:
creating a first and a second picture of the object (2) illuminated by the X-ray source (4; 60) using a sensor (8; 66) which is located, in relation to the X-ray source (4; 60), behind the object (2) in a preferential direction (9; 68) defined by a relative position of the object (2) and of the sensor (8; 66),
wherein a distance in the preferential direction (9; 68) between the X-ray source (4; 60) and the object (2) is different in the first picture and in the second picture,
wherein a further X-ray source (30; 62) for illuminating the object (2) is provided in front of the object (2) in a further preferential direction (34; 70),
wherein the further X-ray source (30; 62) or the object (2) is moved in the further preferential direction (34; 70) so as to change a further distance in the further preferential direction (34; 70) between the further X-ray source (30; 62) and the object (2); and
determining, from the change in the distance between the first and second pictures, an angle of rotation which describes a rotation of the X-ray source (4; 60) in relation to the object (2) which corresponds to a change in the perspective from which the object (2) is illuminated and imaged on the sensor (8; 66); and
combining the first and second pictures while using the angle of rotation and a computer tomography image processing algorithm in order to acquire the multi-dimensional representation of the object (2),
wherein the preferential direction (9, 68) is perpendicular to the further preferential direction (34; 70), wherein the further preferential direction (34; 70) is defined by the relative positions of the object (2) and of the further sensors (32; 64), and wherein the further X-ray source (30, 70) is arranged in front of the object (2), the method further comprising: creating a third picture while using the further sensor (32, 64), wherein the object (2) is illuminated by the further X-ray source (30; 70), wherein combining comprises combining the first picture, the second picture and the third picture in order to acquire the multi-dimensional representation of the object (2).

15. Computer program having a program code for performing the method as claimed in claim 13 or 14 when the program runs on a computer.

## Revendications

1. Dispositif pour générer une représentation multidimensionnelle d'un objet (2), aux caractéristiques suivantes:
une source de rayons X (4; 60) destinée à éclairer l'objet (2) et une autre source de rayons X (30; 62) destinée à éclairer l'objet (2);
un capteur (8; 66) qui se trouve, par rapport à la source de rayons X (4; 60), dans la direction préférée (9; 68) déterminée par la position relative de l'objet (2) et du capteur (8; 66), derrière l'objet (2), pour générer une première prise de vue et une deuxième prise de vue de l'objet (2),
dans lequel l'autre source de rayons X (30; 62) se trouve, dans une autre direction préférée (34; 70), devant l'objet (2);
un moyen de déplacement destiné à déplacer la source de rayons X (4; 60) ou l'objet (2) pour modifier une distance, dans la direction préférée (9; 68), entre la source de rayons X (4; 60) et l'objet (2) entre la première et la deuxième prise de vue, où le dispositif de déplacement est par ailleurs conçu pour déplacer l'autre source de rayons X (30; 62) dans l'autre direction préférée pour modifier une autre distance, dans l'autre direction préférée (34; 70), entre l'autre source de rayons X (30; 62) et l'objet (2); et
un moyen de combinaison (12) qui est conçu pour déterminer, à partir de la modification de la distance entre la première et la deuxième prise de vue, un angle de rotation qui décrit une rotation de la source de rayons X (4; 60) par rapport à l'objet (2) qui correspond à une modification de la perspective selon laquelle l'objet (2) est éclairé et reproduit sur le capteur (8; 66), et
dans lequel le dispositif de combinaison (12) est par ailleurs configuré pour combiner la première prise de vue et la deuxième prise de vue à l'aide de l'angle de rotation et d'un algorithme de traitement d'image par tomographie par ordinateur pour obtenir la représentation multidimensionnelle de l'objet (2),
dans lequel le capteur (8) est par ailleurs conçu pour générer une troisième prise de vue, dans lequel l'objet (2) est éclairé par l'autre source de rayons X (30), dans lequel le moyen de combinaison (12) est conçu pour combiner la première prise de vue, la deuxième prise de vue et la troisième prise de vue pour obtenir la représentation multidimensionnelle de l'objet (2).

2. Dispositif pour générer une représentation multidimensionnelle d'un objet (2), aux caractéristiques suivantes:
une source de rayons X (4; 60) destinée à éclairer l'objet (2) et une autre source de rayons X (30; 62) destinée à éclairer l'objet (2);
un capteur (8; 66) qui se trouve, par rapport à la source de rayons X (4; 60), dans la direction préférée (9; 68) déterminée par la position relative de l'objet (2) et du capteur (8; 66), derrière l'objet (2), pour générer une première prise de vue et une deuxième prise de vue de l'objet (2),
dans lequel l'autre source de rayons X (30; 62) se trouve, dans une autre direction préférée (34; 70), devant l'objet (2);
un moyen de déplacement destiné à déplacer la source de rayons X (4; 60) ou l'objet (2) pour modifier une distance, dans la direction préférée (9; 68), entre la source de rayons X (4; 60) et l'objet (2) entre la première et la deuxième prise de vue, où le dispositif de déplacement est par ailleurs conçu pour déplacer l'autre source de rayons X (30; 62) ou l'objet (2) dans l'autre direction préférée pour modifier une autre distance, dans l'autre direction préférée (34; 70), entre l'autre source de rayons X (30; 62) et l'objet (2); et
un moyen de combinaison (12) qui est conçu pour déterminer, à partir de la modification de la distance entre la première et la deuxième prise de vue, un angle de rotation qui décrit une rotation de la source de rayons X (4; 60) par rapport à l'objet (2) qui correspond à une modification de la perspective selon laquelle l'objet (2) est éclairé et reproduit sur le capteur (8; 66), et
dans lequel le dispositif de combinaison (12) est par ailleurs configuré pour combiner la première prise de vue et la deuxième prise de vue à l'aide de l'angle de rotation et d'un algorithme de traitement d'image par tomographie par ordinateur pour obtenir la représentation multidimensionnelle de l'objet (2),
dans lequel la direction préférée (9, 68) est perpendiculaire à l'autre direction préférée (34, 70), dans lequel l'autre direction préférée (34; 70) est déterminée par la position relative de l'objet (2) et d'un autre capteur (32; 64), et dans lequel l'autre source de rayons X (30, 70) est disposée devant l'objet (2), dans lequel le dispositif présente par ailleurs la caractéristique suivante:
l'autre capteur (32, 64) qui est conçu pour générer une troisième prise de vue dans laquelle l'objet (2) est éclairé par l'autre source de rayons X (30; 70), et
dans lequel le moyen de combinaison (12) est conçu pour combiner la première prise de vue, la deuxième prise de vue et la troisième prise de vue pour obtenir la représentation multidimensionnelle de l'objet (2).

3. Dispositif selon la revendication 1 ou 2, dans lequel la direction préférée (9) est perpendiculaire à une surface sensible du capteur (8).

4. Dispositif selon l'une des revendications 1 à 3, dans lequel est générée une représentation multidimensionnelle qui contient des informations sur une dimension spatiale additionnelle qui n'est contenue ni dans la première, ni dans la deuxième prise de vue, de sorte que dans la représentation multidimensionnelle soit reproduite au moins une dimension spatiale en plus que dans la première prise de vue et la deuxième prise de vue.

5. Dispositif selon l'une des revendications 1 à 4, dans lequel le moyen de combinaison (12) est conçu pour compenser une modification de mesure provoquée par la modification de la distance, dans la direction préférée (9), entre les prises de vue dans la première prise de vue ou la deuxième prise de vue.

6. Dispositif selon la revendication 5, dans lequel le moyen de combinaison (12) est conçu pour compenser la modification de mesure par un facteur d'échelle qui dépend de la distance dans la direction préférée (9).

7. Dispositif selon l'une des revendications précédentes, à la caractéristique additionnelle suivante:
un moyen de rotation destiné à faire tourner la direction préférée (50a, 50b, 50c) par rapport à l'objet (2) d'un angle prédéterminé.

8. Dispositif selon l'une des revendications précédentes, à la caractéristique additionnelle suivante:
un moyen de translation destiné à effectuer un déplacement relatif (40a, 40b, 40c) de l'objet (2) et de la source de rayons X (4) dans une direction de déplacement latéral qui forme un angle prédéterminé avec la direction préférée (9).

9. Dispositif selon la revendication 8, dans lequel le moyen de translation est conçu pour déplacer la source de rayons X (4) dans une direction perpendiculaire à la direction préférée (9).

10. Dispositif selon la revendication 9, dans lequel le moyen de translation est conçu pour déplacer l'objet (2) dans une direction perpendiculaire à la direction préférée.

11. Dispositif selon la revendication 1,
dans lequel la source de rayons X (60) et l'autre source de rayons X (62) sont disposées selon un angle de 90° l'une par rapport à l'autre,
dans lequel est par ailleurs présent, en plus du capteur (66), un autre capteur (64),
dans lequel le capteur (66) est disposé d'un côté de la source de rayons X (60) opposé par rapport à l'objet (2),
dans lequel l'autre capteur (64) est disposé d'un côté de l'autre source de rayons X (60) opposé par rapport à l'objet (2),
dans lequel la direction préférée (68) est perpendiculaire à l'autre direction préférée (70); et
dans lequel le dispositif de déplacement est un dispositif de déplacement commun destiné à déplacer l'objet (2) dans une direction de déplacement perpendiculaire à la direction préférée (68) et à l'autre direction préférée (70) par rapport à la source de rayons X (60) et au capteur (66) et destiné à le déplacer par rapport à l'autre source de rayons X (62) et l'autre capteur (64) ou, alternativement, pour déplacer un système de détection qui présente la source de rayons X (60) et le capteur (66) et l'autre source de rayons X (62) et l'autre capteur (64) devant des zones à examiner de l'objet (2).

12. Dispositif selon la revendication 1,
dans lequel la direction préférée (9) et l'autre direction préférée (34, 70) sont identiques, et sont déterminées par une orientation relative de l'objet (2) et du capteur (8), et
dans lequel sont utilisées la source de rayons X (4) et l'autre source de rayons X (30) dont les trajets de déplacement sont parallèles de chaque côté de l'objet (2), et
dans lequel la source de rayons X (4) et l'autre source de rayons X (30) sont déplacées de points les plus distants de l'objet (2) à des points (20) les moins distants de l'objet (2) pour obtenir des perspectives selon une plage angulaire d'environ 180°.

13. Procédé mis en oeuvre par ordinateur pour générer une représentation multidimensionnelle d'un objet (2), comprenant le fait de:
générer une première et une deuxième prise de vue de l'objet (2) éclairé par une source de rayons X (4; 60) par un capteur (8; 66) qui se trouve, par rapport à la source de rayons X (4; 60), dans une direction préférée (9; 68) déterminée par la position relative de l'objet (2) et du capteur (8; 66), derrière l'objet (2),
dans lequel une distance dans la direction préférée (9; 68) entre la source de rayons X (4; 60) et l'objet (2) diffère entre la première prise de vue et la deuxième prise de vue,
dans lequel une autre source de rayons X (30; 62) destinée à éclairer l'objet (2) dans une autre direction préférée (34; 70) est prévue devant l'objet (2),
dans lequel l'autre source de rayons X (30; 62) est déplacée dans l'autre direction préférée (34; 70) pour modifier une autre distance dans l'autre direction préférée (34; 70) entre l'autre source de rayons X (30, 62) et l'objet (2); et
déterminer un angle de rotation à partir de la modification de la distance entre la première prise de vue et la deuxième prise de vue qui décrit une rotation de la source de rayons X (4; 60) par rapport à l'objet (2) qui correspond à une modification de la perspective selon laquelle l'objet (2) est éclairé et reproduit sur le capteur (8; 66); et
combiner la première prise de vue et la deuxième prise de vue à l'aide de l'angle de rotation et d'un algorithme de traitement d'image par tomographie par ordinateur pour obtenir la représentation multidimensionnelle de l'objet (2),
générer une troisième prise de vue par le capteur (8), où l'objet (2) est éclairé par l'autre source de rayons X (30), où la combinaison présente le fait de combiner la première prise de vue, la deuxième prise de vue et la troisième prise de vue pour obtenir la représentation multidimensionnelle de l'objet (2).

14. Procédé mis en œuvre par ordinateur pour générer une représentation multidimensionnelle d'un objet (2), comprenant le fait de:
générer une première et une deuxième prise de vue de l'objet (2) éclairé par une source de rayons X (4; 60) par un capteur (8; 66) qui se trouve, par rapport à la source de rayons X (4; 60), dans une direction préférée (9; 68) déterminée par la position relative de l'objet (2) et du capteur (8; 66), derrière l'objet (2),
dans lequel une distance dans la direction préférée (9; 68) entre la source de rayons X (4; 60) et l'objet (2) diffère entre la première prise de vue et la deuxième prise de vue,
dans lequel une autre source de rayons X (30; 62) destinée à éclairer l'objet (2) dans une autre direction préférée (34; 70) est prévue devant l'objet (2),
dans lequel l'autre source de rayons X (30; 62) ou l'objet (2) est déplacée dans l'autre direction préférée (34; 70) pour modifier une autre distance dans l'autre direction préférée (34; 70) entre l'autre source de rayons X (30, 62) et l'objet (2); et
déterminer un angle de rotation à partir de la modification de la distance entre la première prise de vue et la deuxième prise de vue qui décrit une rotation de la source de rayons X (4; 60) par rapport à l'objet (2) qui correspond à une modification de la perspective selon laquelle l'objet (2) est éclairé et reproduit sur le capteur (8; 66); et
combiner la première prise de vue et la deuxième prise de vue à l'aide de l'angle de rotation et d'un algorithme de traitement d'image par tomographie par ordinateur pour obtenir la représentation multidimensionnelle de l'objet (2),
dans lequel la direction préférée (9, 68) est perpendiculaire à l'autre direction préférée (34, 70), dans lequel l'autre direction préférée (34; 70) est déterminée par la position relative de l'objet (2) et d'un autre capteur (32; 64), et dans lequel l'autre source de rayons X (30, 70) est disposée devant l'objet (2), dans lequel le procédé présente par ailleurs le fait de: générer une troisième prise de vue par l'autre capteur (32, 64), dans lequel l'objet (2) est éclairé par l'autre source de rayons X (30; 70), dans lequel la combinaison comporte le fait de combiner la première prise de vue, la deuxième prise de vue et la troisième prise de vue pour obtenir la représentation multidimensionnelle de l'objet (2).

15. Programme d'ordinateur avec un code de programme pour réaliser le procédé selon la revendication 13 ou 14 lorsque le programme est exécuté sur un ordinateur.
